(19) Europäisches Patentamt
European Patent Office
Office européen des brevets

(11) Veröffentlichungsnummer: **0 044 954**
**B1**

(12) # EUROPÄISCHE PATENTSCHRIFT

(45) Veröffentlichungstag der Patentschrift: **17.04.85**

(51) Int. Cl.[4]: **C 07 H 15/24,** A 61 K 31/70 // C07C46/00, C07C39/14, C07D317/72, C07D339/04

(21) Anmeldenummer: **81104979.0**

(22) Anmeldetag: **26.06.81**

(54) **Neue Anthracyclinglycoside, ihre Herstellung und pharmazeutische Präparate.**

(30) Priorität: **18.07.80 GB 8023498**
**26.05.81 GB 8116053**

(43) Veröffentlichungstag der Anmeldung: **03.02.82 Patentblatt 82/5**

(45) Bekanntmachung des Hinweises auf die Patenterteilung: **17.04.85 Patentblatt 85/16**

(84) Benannte Vertragsstaaten: **AT BE CH DE FR GB IT LI LU NL SE**

(56) Entgegenhaltungen:
**FR - A - 2 355 028**
**FR - A - 2 424 285**
**GB - A - 2 048 245**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Broadhurst, Michael John, 14 Angells Meadow, Ashwell, Baldock, Herts (GB)**
Erfinder: **Hassall, Cedric Herbert, 6 Ashcroft Close, Harpenden, Herts (GB)**
Erfinder: **Thomas, Gareth John, 62 Crosslands, Caddington, Luton, Bedfordshire (GB)**

(74) Vertreter: **Grossner, Lutz, Dr. et al, Grenzacher Strasse 124 Postfach 3255, CH-4002 Basel (CH)**

**EP 0 044 954 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue Anthracyclinglykoside, ein Verfahren zu deren Herstellung und pharmazeutische Präparate, die diese Glykoside enthalten.

Die erfindungsgemäßen neuen Anthracyclinglykoside besitzen die allgemeine Formel

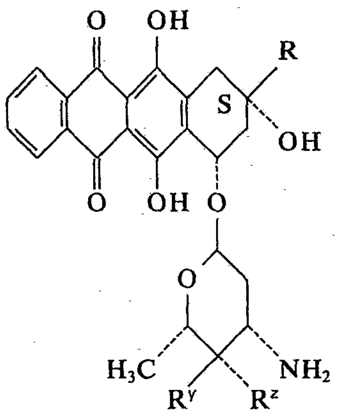

(I)

worin R nieder-Alkyl oder Carboxy oder eine Gruppe der Formel

$$—(CH_2)_n—OY \quad (a)$$

darstellt, wobei n 1 oder 2, Y Wasserstoff oder Alkyl und $R^y$ und $R^z$ jeweils Wasserstoff oder einer der Reste $R^y$ und $R^z$ Wasserstoff und der andere Hydroxy bedeuten.

Die Erfindung umfaßt ebenfalls pharmazeutisch anwendbare Säureadditionssalze der Verbindungen der Formel I.

Eine bevorzugte Gruppe von Verbindungen der Formel I sind diejenigen, in denen R nieder-Alkyl oder eine Gruppe der Formel (a), wobei Y Wasserstoff ist, darstellt. Ebenfalls bevorzugt sind Verbindungen der Formel I, in denen ein Rest $R^y$ und $R^z$ Wasserstoff und der andere Hydroxy ist.

Beispiele bevorzugter Verbindungen der Formel I sind:

(1 S)-cis-1-[(3-Amino-2,3,6-trideoxy-α-L-lyxohexopyranosyl)oxy]-1,2,3,4,6,11-hexahydro-3,5,12-trihydroxy-3-hydroxymethyl-6,11-dioxonaphthacen,
(1 S)-cis-1-[(-3-Amino-2,3,6-trideoxy-α-L-lyxohexopyranosyl)oxy]-1,2,3,4,6,11-hexahydro-3,5,12-trihydroxy-3-methyl-6,11-dioxonaphthacen,
(1 S)-cis-1-[(3-Amino-2,3,6-trideoxy-α-L-arabinohexopyranosyl)oxy]-1,2,3,4,6,11-hexahydro-3,5,12-trihydroxy-3-methyl-6,11-dioxonaphthacen,
(1 S)-cis-1-[(3-Amino-2,3,6-trideoxy-α-L-arabinohexopyranosyl)oxy]-1,2,3,4,6,11-hexahydro-3,5,12-trihydroxy-3-hydroxymethyl-6,11-dioxonaphthacen und
(1 S)-cis-1-[(3-Amino-2,3,6-trideoxy-α-L-lyxohexopyranosyl)oxy]-3-äthyl-1,2,3,4,6,11-hexahydro-3,5,12-trihydroxy-6,11-dioxonaphthacen.

Die Verbindungen der Formel I und ihre pharmazeutisch anwendbaren Säureadditionssalze werden erfindungsgemäß dadurch hergestellt, daß man eine Verbindung der Formel

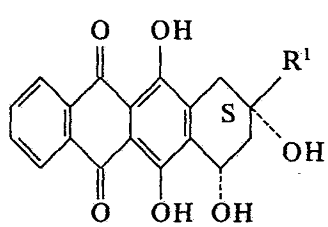

(II)

worin $R^1$ nieder-Alkyl oder eine veresterte Carboxygruppe oder eine Gruppe der Formel

$$—(CH_2)_n—OY' \quad (a')$$

worin n die obige Bedeutung hat und Y' Alkyl oder Acyl ist, darstellt,
mit einer Verbindung der Formel

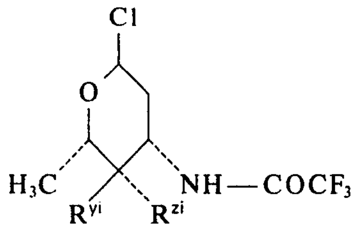

(III)

worin $R^{yi}$ und $R^{zi}$ jeweils Wasserstoff oder einer der Reste $R^{yi}$ und $R^{zi}$ Wasserstoff und der andere p-Nitrobenzoyloxy darstellen,
umsetzt, die Schutzgruppe(n) aus dem Reaktionsprodukt abspaltet und gewünschtenfalls die erhaltene Verbindung der Formel I in ein pharmazeutisch anwendbares Säureadditionssalz überführt.

Der hier verwendete Ausdruck »nieder-Alkyl« bezieht sich auf eine geradkettige oder verzweigte Alkylgruppe mit vorzugsweise 1–6 C-Atomen, wie Methyl, Äthyl, Propyl, Isopropyl, Butyl, t-Butyl, Pentyl und Hexyl. Eine veresterte Carboxygruppe kann eine Alkoxycarbonylgruppe (z. B. Methoxycarbonyl oder Äthoxycarbonyl), eine Aryloxycarbonylgruppe (z. B. Phenoxycarbonyl) oder eine Aralkoxycarbonylgruppe (z. B. Benzyloxycarbonyl) sein. Methoxycarbonyl ist die bevorzugte Alkoxycarbonylgruppe. Eine Acylgruppe oder der Acylrest einer Acyloxygruppe kann sich von einer Alkancarbonsäure (z. B. Essigsäure und Propionsäure), einer aromatischen Carbonsäure (z. B. Benzoesäure) oder einer araliphatischen Carbonsäure (z. B. Phenylessigsäure) ableiten. Eine Arylgruppe kann beispielsweise Phenyl, substituiertes Phenyl (z. B. Methoxyphenyl) oder Pyridyl sein.

Die Umsetzung einer Verbindung der Formel II mit einer Verbindung der Formel III gemäß dem erfindungsgemäßen Verfahren kann in an sich bekannter Weise durchgeführt werden. In einer bevorzugten Ausführungsform wird die Reaktion in einem inerten organischen Lösungsmittel und in Gegenwart eines löslichen Silbersalzes durchgeführt. Beispiele inerter organischer Lösungsmittel sind Dichlormethan, Dimethylformamid und Tetrahydrofuran, welch letzteres bevorzugt ist. Vorzugsweise wird die Reaktion bei niedriger Temperatur, z. B. bei −5°C ausgeführt.

Die Schutzgruppe(n) werden dann aus dem Reaktionsprodukt der allgemeinen Formel

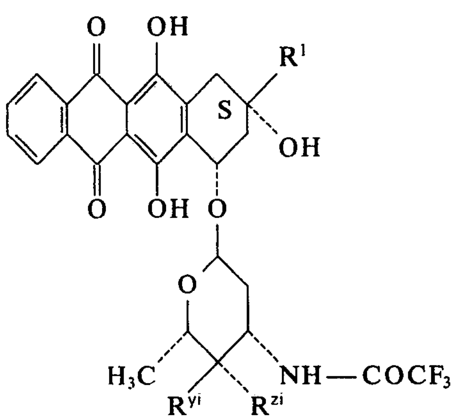

(IV)

worin $R^1$, $R^{yi}$ und $R^{zi}$ die obige Bedeutung haben,
abgespalten.

Die Abspaltung der Schutzgruppe(n) aus der Verbindung der Formel IV kann in an sich bekannter Weise, beispielsweise durch Behandlung mit wäßrigem Alkali wie wäßriger Natronlauge bewerkstelligt werden. Falls zwei Schutzgruppen in der Verbindung der Formel IV anwesend sind, kann die Abspaltung solcher Gruppen in einem oder zwei Schritten durch entsprechende Wahl der Abspaltungsbedingungen ausgeführt werden. Beispielsweise kann in einem zweistufigen Verfahren zunächst eine p-Nitrobenzoylschutzgruppe abgespalten werden, worauf dann die Abspaltung einer Trifluoracetylschutzgruppe folgt.

Die Verbindungen der Formel I können in pharmazeutisch anwendbare Säureadditionssalze durch Behandlung mit pharmazeutisch anwendbaren anorganischen Säuren (z. B. Salzsäure, Bromwasserstoffsäure, Schwefelsäure oder Phosphorsäure) oder mit pharmazeutisch anwendbaren organischen Säuren (z. B. Essigsäure, Weinsäure, Zitronensäure, Fumarsäure, Maleinsäure, Äpfelsäure, Methansulfonsäure oder Toluol-4-sulfonsäure) erreicht werden.

Die Verbindungen der Formel I enthalten zwei asymmetrische C-Atome im Aglyconteil und die Erfindung betrifft die (1 S)-cis-Verbindungen.

Die als Ausgangsmaterialien im erfindungsgemäßen Verfahren verwendeten Verbindungen der Formel II können aus Verbindungen der Formel

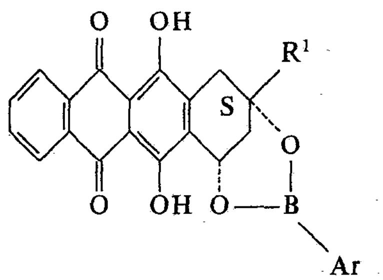

(V)

worin $R^1$ die obige Bedeutung hat und Ar eine Arylgruppe darstellt, durch Umesterung mit einem 1,3-Diol hergestellt werden.

Diese Umesterung kann zweckmäßig durch Umsetzung einer Verbindung der Formel V mit überschüssigem 1,3-Diol in Gegenwart einer Säure ausgeführt werden. Ein besonders bevorzugtes 1,3-Diol ist 2-Methyl-2,4-pentandiol. Bevorzugte Säuren für diese Reaktion sind niedere Alkancarbonsäuren wie Essigsäure. Die Reaktion wird zweckmäßig in Gegenwart eines inerten organischen Lösungsmittels (z.B. in einem halogenierten Kohlenwasserstoff wie Dichlormethan) und bei etwa Raumtemperatur durchgeführt.

Die Verbindungen der Formel V können durch Deacylierung einer Verbindung der Formel

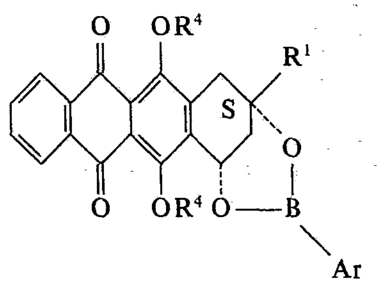

(VI)

worin $R^1$ und Ar die obige Bedeutung haben und $R^4$ einen Acylrest darstellt, hergestellt werden.

Die Deacylierung einer Verbindung der Formel VI wird zweckmäßig mittels Bortrichlorid in einem inerten organischen Lösungsmittel (z.B. einem halogenierten Kohlenwasserstoff wie Dichlormethan) und bei niedriger Temperatur (z.B. bei etwa −10°C) durchgeführt. Die Deacylierung kann auch durch Behandlung mit einer wäßrigen Säure oder Base unter konventionellen Bedingungen ausgeführt werden.

Die Verbindungen der Formel VI können durch Oxidation einer Verbindung der Formel

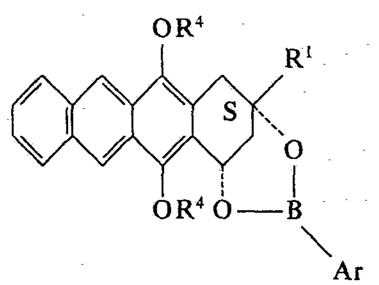

(VII)

worin $R^1$, $R^4$ und Ar die obige Bedeutung haben, mit einem Chrom(VI)-Oxidationsmittel unter wasserfreien Bedingungen hergestellt werden.

Ein bevorzugtes Chrom(VI)-Oxidationsmittel für die vorstehende Oxidation ist Chromtrioxid. In einer bevorzugten Ausführungsform wird diese Oxidation in Gegenwart eines Gemisches einer geeigneten wasserfreien Carbonsäure und dem entsprechenden Anhydrid (z.B. einem Gemisch von Eisessig und Acetanhydrid) ausgeführt. Die Oxidation kann bei einer Temperatur zwischen etwa Raumtemperatur und etwa 60°C durchgeführt werden, vorzugsweise wird sie bei etwa Raumtemperatur ausgeführt.

Die Verbindungen der Formel VII können durch katalytische Hydrierung einer Verbindung der Formel

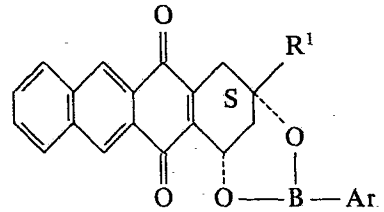

(VIII)

worin $R^1$ und Ar die obige Bedeutung haben,
unter acylierenden Bedingungen hergestellt werden.

Geeignete Katalysatoren, die bei der katalytischen Hydrierung einer Verbindung der Formel VIII verwendet werden können, sind Edelmetall-Katalysatoren wie beispielsweise Palladium, Platin, Ruthenium und Rhodium. Der Katalysator kann auf einem geeigneten Träger (z. B. Palladium auf Kohle) aufgebracht sein. Die acylierenden Bedingungen werden dadurch hergestellt, daß man die katalytische Hydrierung in Gegenwart eines geeigneten Acylierungsmittels, vorzugsweise eines Carbonsäureanhydrids wie Acetanhydrid, durchführt. Eine tertiäre organische Base ist im Reaktionsgemisch als säurebindendes Mittel zweckmäßigerweise anwesend. Geeignete tertiäre organische Basen sind beispielsweise Tri(nieder-alkyl)amine wie Triäthylamin, Pyridin und Collidin, wovon Pyridin bevorzugt ist. Die katalytische Hydrierung wird vorteilhafterweise bei Raumtemperatur und Atmosphärendruck durchgeführt.

Die Verbindungen der Formel VIII können durch Umsetzung einer Verbindung der Formel

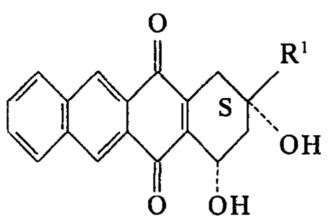

(IX)

worin $R^1$ die obige Bedeutung hat,
mit einer aromatischen Boronsäure hergestellt werden.

Die Umsetzung einer Verbindung der Formel IX mit einer aromatischen Boronsäure wird vorzugsweise in einem inerten organischen Lösungsmittel vorgenommen. Bevorzugte Lösungsmittel sind aromatische Kohlenwasserstoffe wie Benzol, Toluol oder Xylol. Eine bevorzugte aromatische Boronsäure für diese Reaktion ist Benzolboronsäure. Jedoch können auch andere aromatische Boronsäuren wie Toluolboronsäure, Xylolboronsäure, Methoxybenzolboronsäure und Nitrobenzolboronsäure und Pyridinboronsäure angewendet werden. Zweckmäßig wird die Reaktion in Gegenwart einer katalytischen Menge einer Carbonsäure, vorzugsweise einer niederen Alkancarbonsäure wie Essigsäure oder Propionsäure durchgeführt. Vorteilhafterweise wird die Reaktion bei erhöhter Temperatur, zweckmäßig bei Rückflußtemperatur des Reaktionsgemisches ausgeführt.

Je nach den Reaktionsbedingungen bei der Herstellung der Verbindungen der Formel II kann eine Acyloxygruppe zu einer Hydroxygruppe hydrolysiert werden. Wenn das eintritt, kann die Acyloxygruppe in an sich bekannter Weise nach dem betreffenden Reaktionsschritt oder später an geeigneter Stelle wieder eingeführt werden.

Die Verbindungen der Formel IX können wie in nachfolgendem Reaktionsschema I angegeben hergestellt werden, wobei $R^1$ die obige Bedeutung hat und $R^5$ eine Acyloxygruppe darstellt.

Formelschema I

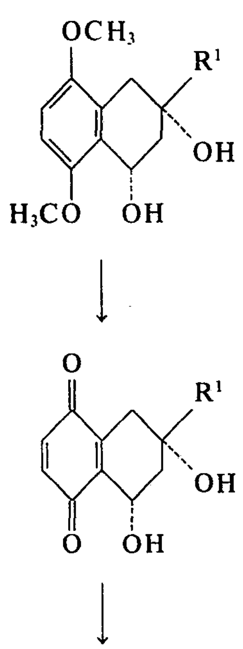

(X)

(XI)

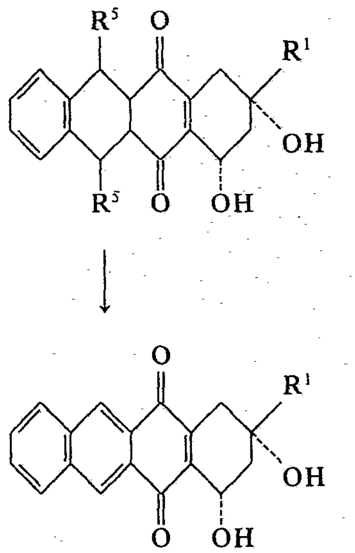

(XII)

(IX)

In Formelschema I wird eine Verbindung der Formel X in eine Verbindung der Formel XI durch Behandlung mit Ammoniumcernitrat umgewandelt. Diese Reaktion wird zweckmäßig in einem Gemisch von Wasser und einem Wasser mischbaren organischen Lösungsmittel (z. B. Acetonitril) und bei etwa Raumtemperatur ausgeführt.

Eine Verbindung der Formel XI wird dann in eine Verbindung der Formel XII durch Umsetzung mit einer trans-Verbindung der allgemeinen Formel

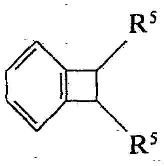

(XIII)

worin $R^5$ die obige Bedeutung hat,
umgewandelt. Diese Reaktion wird zweckmäßig in einem inerten organischen Lösungsmittel, insbesondere einem aromatischen Kohlenwasserstoff wie Benzol, Toluol oder Xylol durchgeführt. Vorzugsweise wird die Reaktion bei erhöhter Temperatur, am besten bei Rückflußtemperatur des Reaktionsgemisches, ausgeführt. Gewünschtenfalls kann man unter Inertgasatmosphäre (wie Stickstoff oder Argon) arbeiten.

Aus der Verbindung der Formel XII werden dann 2 Mole Carbonsäure $R^5H$ durch Erhitzen oder Behandlung mit einer Base eliminiert, wobei man eine Verbindung der Formel IX erhält. Das Erhitzen einer Verbindung der Formel XII wird vorzugsweise in einem inerten organischen Lösungsmittel wie einem aromatischen Kohlenwasserstoff, z. B. Benzol, Toluol oder Xylol, ausgeführt. Zweckmäßig erhitzt man bis zur Rückflußtemperatur des Reaktionsgemisches. Gewünschtenfalls kann das Erhitzen unter Inertgasatmosphäre, z. B. unter Stickstoff oder Argon, durchgeführt werden. Vorzugsweise wird eine Verbindung der Formel XII in situ erhitzt, d. h. ohne daß sie aus dem Reaktionsmedium, in dem sie hergestellt wird, isoliert wird. Bei der Behandlung einer Verbindung der Formel XII mit einer Base kann eine anorganische oder eine organische Base verwendet werden. Vorzugsweise führt man die Behandlung mittels einer anorganischen Base, insbesondere eines Alkalimetallhydroxids wie Natriumhydroxid oder Kaliumhydroxid, in einem niederen Alkanol, z. B. Methanol oder Äthanol, durch. Diese Behandlung wird zweckmäßigerweise bei etwa Raumtemperatur durchgeführt.

Die Verbindungen der Formel X können wiederum aus cis/trans-Verbindungen der Formel X erhalten werden. Eine derartige cis/trans-Verbindung kann mit einer aromatischen Boronsäure behandelt werden, wobei man ein Gemisch des cis-Boronsäureesters und unverändertem trans-Diol erhält. Dieses Gemisch kann getrennt werden und der cis-Boronsäureester kann in das cis-Diol übergeführt werden. Die Behandlung mit einer aromatischen Boronsäure, z. B. einer der oben erwähnten, insbesondere Benzolboronsäure, wird zweckmäßig in einem inerten organischen Lösungsmittel wie Äthylacetat bei erhöhter Temperatur, zweckmäßig bei Rückflußtemperatur und gewünschtenfalls unter Inertgas wie Stickstoff oder Argon durchgeführt. Die Trennung des cis-Boronsäureesters und des trans-Diols kann durch Chromatographie, zweckmäßigerweise auf Silicagel, durchgeführt werden. Der cis-Boronsäureester wird in das cis-Diol zweckmäßig durch Behandeln mit einer Säure, vorzugsweise einer Carbonsäure wie Essigsäure in Gegenwart eines Überschusses eines 1,3-Diols wie 2-Methyl-2,4-pentandiol umgewandelt. Die Behandlung wird zweckmäßig in einem inerten organischen Lösungsmittel, vorzugsweise einem halogenierten Kohlenwasserstoff wie Dichlormethan und bei Raumtemperatur vorgenommen.

Ein trans-Diol kann in den entsprechenden cis-Boronsäureester umgewandelt werden, der wiederum in das cis-Diol übergeführt werden kann. Die Umwandlung des trans-Diols in den cis-Boronsäureester kann durch Behandlung mit einer aromatischen Boronsäure wie einer der früher erwähnten Boronsäuren, vorzugsweise Benzolboronsäure, in Gegenwart einer organischen Sulfonsäure, vorzugsweise einer aromatischen Sulfonsäure wie Toluol-4-sulfonsäure durchgeführt werden. Vorteilhafterweise führt man die Reaktion in einem organischen Lösungsmittel, vorzugsweise einem aromatischen Kohlenwasserstoff wie Benzol, bei etwa Raumtemperatur durch. Der erhaltene cis-Boronsäureester kann dann in das cis-Diol in der früher beschriebenen Weise umgewandelt werden.

Alternativ können Verbindungen der Formel VIII wie in Formelschema II angegeben hergestellt werden, wobei $R^1$ und Ar die obige Bedeutung haben.

Formelschema II

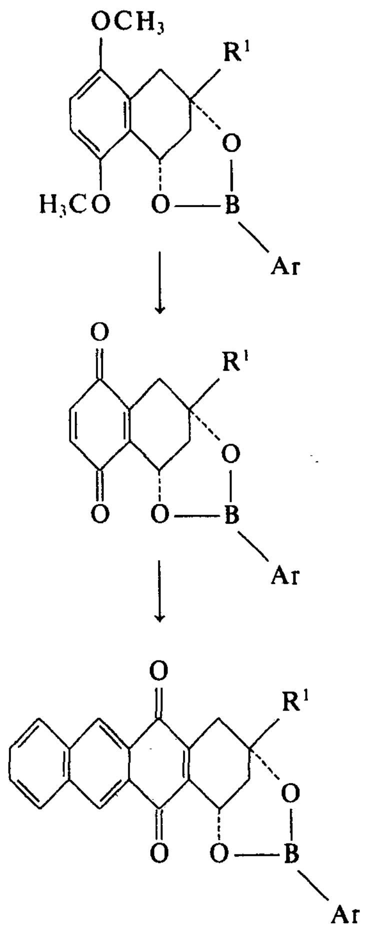

(XIV)

(XV)

(VIII)

Gemäß Formelschema II wird eine Verbindung der Formel XIV, die wie früher beschrieben aus einer cis/trans-Verbindung der Formel X erhalten werden kann, in eine Verbindung der Formel XV durch Behandlung mit Ammoniumcernitrat umgewandelt. Diese Behandlung wird vorteilhaft in Gegenwart eines Gemisches von Wasser und einem mit Wasser mischbaren inerten organischen Lösungsmittel wie Acetonitril und bei etwa Raumtemperatur durchgeführt.

Die Verbindung der Formel XV wird dann in eine Verbindung der Formel VIII durch Umsetzung mit einer trans-Verbindung der Formel XIII übergeführt. Diese Reaktion wird zweckmäßig in einem inerten organischen Lösungsmittel, insbesondere einem aromatischen Kohlenwasserstoff wie Benzol, Toluol oder Xylol und bei erhöhter Temperatur insbesondere bei Rückflußtemperatur des Reaktionsgemisches ausgeführt. Gewünschtenfalls kann man unter Inertgas, z.B. unter Stickstoff oder Argon, arbeiten.

Cis/trans-Verbindungen der Formel X, worin $R^1$ eine Gruppe der Formel (a′) bedeutet und worin n 1 ist, können beispielsweise dadurch hergestellt werden, daß man eine Verbindung der Formel

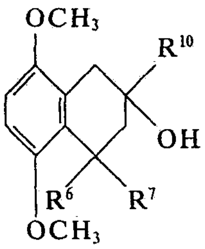

(XVI)

worin $R^{10}$ eine veresterte Carboxygruppe und $R^6$ und $R^7$ gemeinsam eine Alkylendithiogruppe, insbesondere die Äthylendithiogruppe bedeuten,
in an sich bekannter Weise zu einer Verbindung der Formel

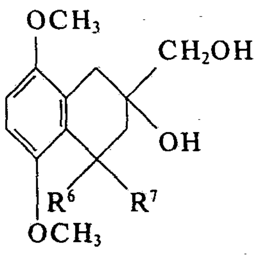

(XVII)

worin $R^6$ und $R^7$ die obige Bedeutung haben,
reduziert, die Verbindung der Formel XVII entsprechend veräthert oder acyliert, die erhaltene Verbindung der Formel

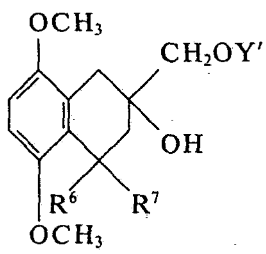

(XVIII)

worin $R^6$, $R^7$ und Y' die obige Bedeutung haben,
mit einem Quecksilber(II)-salz behandelt und die erhaltene Verbindung der Formel

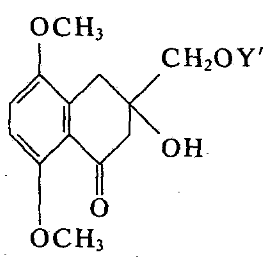

(XIX)

worin Y' die obige Bedeutung hat,
reduziert.

Die Reduktion einer Verbindung der Formel XVI kann in an sich bekannter Weise vorgenommen werden, beispielsweise mittels eines Alkalimetallborhydrids wie Natriumborhydrid, in einem inerten organischen Lösungsmittel wie Tetrahydrofuran.

Die Verätherung einer Verbindung der Formel XVII kann in an sich bekannter Weise beispielsweise mit einem Alkylhalid (z. B. Methyljodid) in Gegenwart einer Base (z. B. Natriumhydrid) und in einem inerten organischen Lösungsmittel wie Dioxan, Tetrahydrofuran oder 1,2-Dimethoxyäthan, durchgeführt werden. Die Acylierung einer Verbindung der Formel XVII kann ebenfalls in an sich bekannter Weise durchgeführt werden.

Die Behandlung einer Verbindung der Formel XVIII mit einem Quecksilber(II)-salz wird vorzugsweise mittels eines Gemisches von Quecksilber(II)chlorid und Quecksilber(II)oxid durchgeführt. Zweckmäßigerweise führt man die Reaktion in einem Wasser mischbaren inerten organischen Lösungsmittel wie einem Alkanol (z. B. Methanol oder Äthanol), Tetrahydrofuran oder einem Gemisch derartiger Lösungsmittel, die auch Wasser enthalten können, durch. Die Behandlung wird vorzugsweise bei Raumtemperatur vorgenommen.

Eine Verbindung der Formel XIX wird anschließend in an sich bekannter Weise zu der gewünschten cis/trans-Verbindung der Formel X reduziert, in der $R^1$ eine Gruppe der Formel (a') mit $n = 1$ bedeutet. Diese Reduktion wird zweckmäßig mittels Alkalimetallborhydriden, vorzugsweise Natriumborhydrid in einem üblichen inerten organischen Lösungsmittel wie Tetrahydrofuran durchgeführt. Zweckmäßig wird die Reduktion bei Raumtemperatur ausgeführt. Gewünschtenfalls kann man unter Inertgasatmosphäre wie Stickstoff oder Argon arbeiten.

Cis/trans-Verbindungen der Formel X, in denen $R^1$ eine Gruppe der Formel (a') mit $n = 2$ bedeutet, können beispielsweise dadurch hergestellt werden, daß man zunächst eine Verbindung der Formel XVII in eine Verbindung der Formel

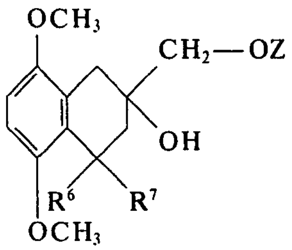

(XX)

worin $R^6$ und $R^7$ die obige Bedeutung haben und Z nieder-Alkylsulfonyl oder Arylsulfonyl darstellt, umwandelt. Diese Umwandlung kann in an sich bekannter Weise beispielsweise durch Umsetzung mit einem nieder-Alkylsulfonylchlorid (z. B. Methansulfonylchlorid) oder vorzugsweise mit einem Arylsulfonylchlorid (z. B. Toluol-4-sulfonylchlorid) in Gegenwart einer geeigneten Base (z. B. ein tertiäres Amin wie Pyridin oder 4-Dimethylaminopyridin) und bei niedriger Temperatur (z. B. bei 0—5°C) vorgenommen werden.

Im nächsten Schritt wird eine Verbindung der Formel XX mit einem Alkalimetallcyanid behandelt, wobei man eine Verbindung der Formel

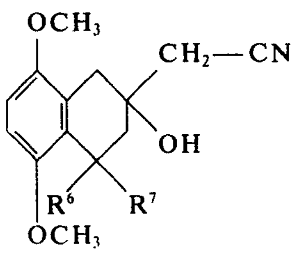

(XXI)

worin $R^6$ und $R^7$ die obige Bedeutung haben,
erhält. Diese Behandlung wird in an sich bekannter Weise ausgeführt, beispielsweise mittels Kaliumcyanid in wäßrigem Dimethylsulfoxid oder Dimethylformamid.

Eine Verbindung der Formel XXI wird dann zu einer Verbindung der Formel

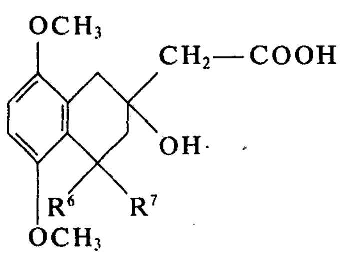

(XXII)

worin $R^6$ und $R^7$ die obige Bedeutung haben,
hydrolysiert. Die Hydrolyse wird in für die Hydrolyse von Nitrilen zu den entsprechenden Säuren an sich bekannter Weise ausgeführt, beispielsweise mittels Alkalimetallhydroxiden wie Kaliumhydroxid in wäßrigem niederen Alkanol wie wäßrigem Äthanol.

Eine Verbindung der Formel XXII wird dann zu einer Verbindung der Formel

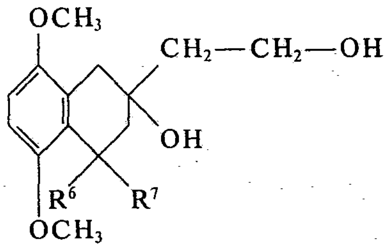

(XXIII)

worin $R^6$ und $R^7$ die obige Bedeutung haben, reduziert. Diese Reduktion kann in für die Reduktion von Carbonsäuren zu entsprechenden Alkoholen an sich bekannter Weise ausgeführt werden. Beispielsweise kann man die Reduktion mit einem Alkalimetallaluminiumhydrid (z. B. Lithiumaluminiumhydrid) in einem inerten organischen Lösungsmittel (z. B. Tetrahydrofuran oder Dioxan) durchführen. Die Reduktion kann aber auch mittels Diboran ausgeführt werden. In gewissen Fällen kann es von Vorteil sein, eine Verbindung der Formel XXII zu einen Ester (z. B. den Methylester) vor der Reduktion zu überführen.

Anschließend wird eine Verbindung der Formel XXIII veräthert oder acyliert, mit einem Quecksilber(II)-salz behandelt und in Analogie zu der früher beschriebenen Weise reduziert, wobei die gewünschte cis/trans-Verbindung der Formel X, worin $R^1$ eine Gruppe der Formel (a') mit $n = 2$ darstellt, erhalten wird.

Cis/trans-Verbindungen der Formel X mit R = Methyl können beispielsweise dadurch hergestellt werden, daß man eine Verbindung der Formel XX mit einem Alkalimetallaluminiumhydrid wie Lithiumaluminiumhydrid in an sich bekannter Weise reduziert und nachfolgend mit einem Quecksilber(II)-salz behandelt und reduziert. Cis/trans-Verbindungen der Formel X, in denen $R^1$ eine andere nieder-Alkylgruppe darstellt, können ähnlich aus entsprechenden $\omega$-(nieder-Alkylsulfonyloxy oder Arylsulfonyloxy)-nieder-Alkyl-Verbindungen erhalten werden. Beispielsweise kann aus einem von einer Verbindung der Formel XXIII abgeleiteten Alkyl- oder Arylsulfonat eine cis/trans-Verbindung der Formel X erhalten werden, in der $R^1$ eine Äthylgruppe darstellt. Andererseits kann dieses nieder-Alkylsulfonat oder Arylsulfonat einer Kettenverlängerung nach dem vorher beschriebenen Verfahren (z. B. via Nitril, Säure und Alkohol) unterworfen werden. Diese Kettenverlängerung kann natürlich bei Bedarf wiederholt werden.

Andererseits können cis/trans-Verbindungen der Formel X mit R = Äthyl, beispielsweise wie in Formelschema III hergestellt werden, wobei $R^6$, $R^7$, $R^{10}$ und Z die früher angegebene Bedeutung haben.

Formelschema III

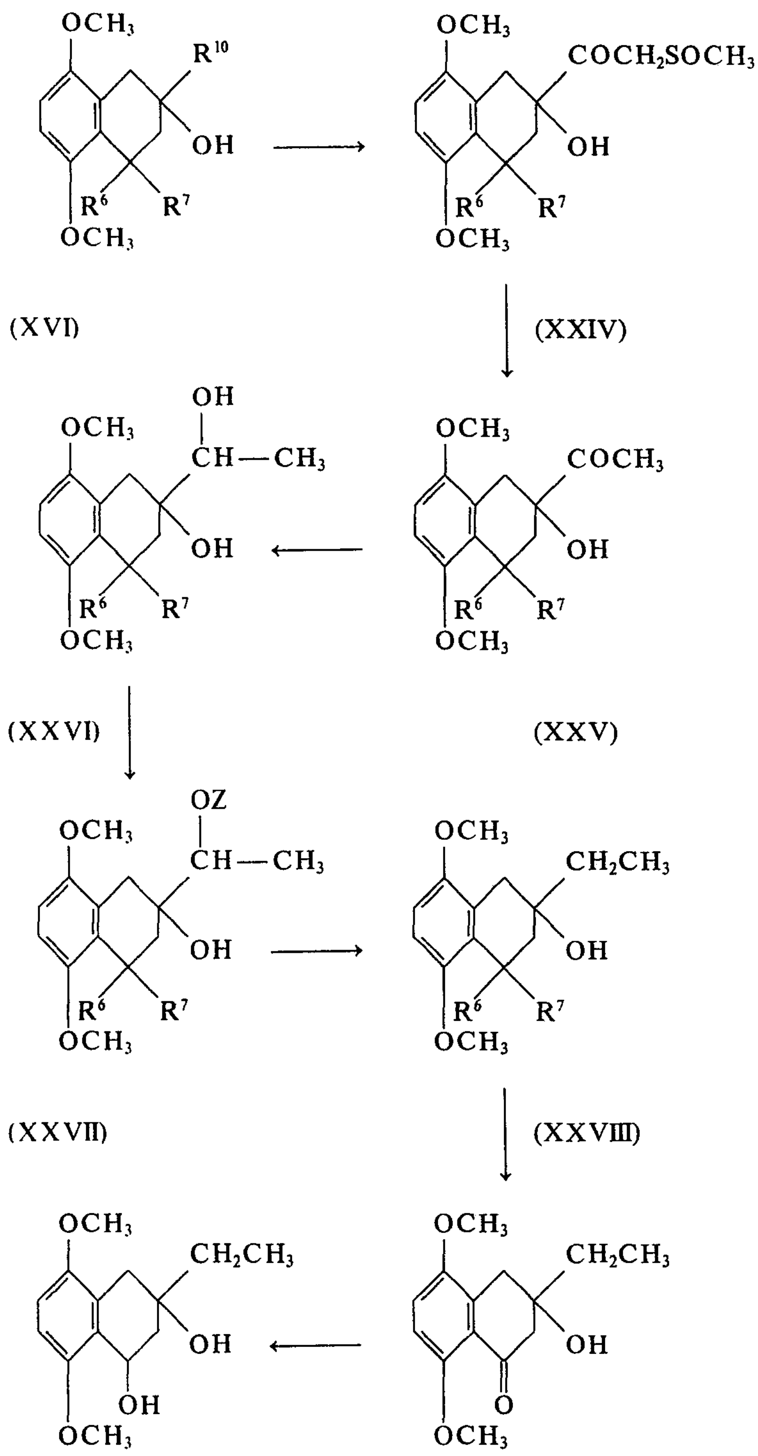

(XVI) (XXIV) (XXV) (XXVI) (XXVII) (XXVIII) (XXIX) (XXX)

Gemäß Formelschema III wird eine Verbindung der Formel XVI in eine Verbindung der Formel XXIV durch Behandlung mit einem Alkalimetallsalz von Dimethylsulfoxid übergeführt. Diese Reaktion wird vorzugsweise mit dem Natriumsalz von Dimethylsulfoxid und in einem inerten organischen Lösungsmittel, z. B. Tetrahydrofuran bei etwa 0°C durchgeführt.

Eine Verbindung der Formel XXIV wird dann durch Behandlung mit einem Aluminiumamalgam in eine Verbindung der Formel XXV übergeführt. Diese Reaktion wird zweckmäßig in Gegenwart eines inerten Lösungsmittels, z. B. wäßrigem Tetrahydrofuran, bei einer Temperatur zwischen etwa 10 und 20°C durchgeführt.

Anschließend wird eine Verbindung der Formel XXV in an sich bekannter Weise zu einer Verbindung

der Formel XXVI reduziert, beispielsweise mittels eines Alkalimetallborhydrids wie Natriumborhydrid in einem inerten organischen Lösungsmittel wie Tetrahydrofuran.

Im nächsten Schritt wird eine Verbindung der Formel XXVI in an sich bekannter Weise in einer Verbindung der Formel XXVII übergeführt, beispielsweise durch Umsetzung mit einem niederen Alkylsulfonylchlorid, z. B. Methansulfonylchlorid, in Gegenwart einer geeigneten Base, z. B. einem tertiären Amin wie Pyridin, und bei niedriger Temperatur, z. B. bei 0–5°C.

Die Verbindung der Formel XVII wird dann durch Reduktion mit einem Alkalimetallaluminiumhydrid wie Lithiumaluminiumhydrid in an sich bekannter Weise in eine Verbindung der Formel XXVIII übergeführt.

Eine Verbindung der Formel XXVIII wird anschließend wie oben im Zusammenhang mit der Umwandlung einer Verbindung der Formel XVIII in eine Verbindung der Formel XIX durch Behandlung mit einem Quecksilber(II)-salz in eine Verbindung der Formel XXIX übergeführt.

Schließlich wird eine Verbindung der Formel XXIX in eine cis/trans-Verbindung der Formel XXX durch Reduktion in Analogie zu den oben für die Reduktion einer Verbindung der Formel XIX beschriebenen Verfahren übergeführt.

Die Verbindungen der Formel XVI können beispielsweise dadurch hergestellt werden, daß man zunächst eine Verbindung der Formel

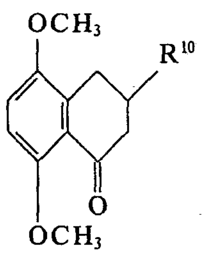

(XXXI)

worin $R^{10}$ die obige Bedeutung hat,
in geeigneter Weise zu einer Verbindung der Formel

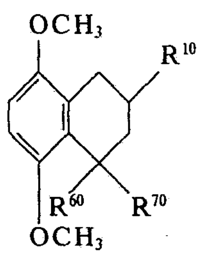

(XXXII)

worin $R^{10}$ die obige Bedeutung hat und $R^{60}$ und $R^{70}$ zusammen eine Alkylendioxy, insbesondere Äthylendioxygruppe darstellen,
ketalisiert. Die Ketalisierung einer Verbindung der Formel XXXI kann in für die Ketalisierung von Oxogruppen an sich bekannter Weise durchgeführt werden. Beispielsweise mit einem geeigneten Alkohol in Gegenwart von Toluol-4-sulfonsäure und in Gegenwart eines geeigneten inerten organischen Lösungsmittels, wie einem aromatischen Kohlenwasserstoff, z. B. Benzol oder Toluol und bei erhöhter Temperatur, z. B. bei Rückflußtemperatur des Reaktionsgemisches.

Eine Verbindung der Formel XXXII wird dann in eine Verbindung der Formel

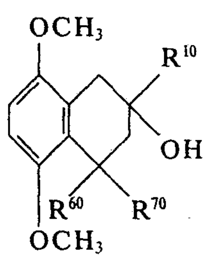

(XXXIII)

worin $R^{10}$, $R^{60}$ und $R^{70}$ die obige Bedeutung haben,
übergeführt.

Diese Überführung wird dadurch vorgenommen, daß man zunächst das Lithiumenolat einer Verbindung der Formel XXXII herstellt und dieses dann entweder mit Diperoxooxohexamethylphosphoramidomolybdän(IV)pyridin ($MoO_5$ · py · HPMT) oder mit Sauerstoff in Gegenwart eines Trialkylphosphits behandelt.

Die Herstellung des Lithiumenolats einer Verbindung der Formel XXXII kann in an sich bekannter Weise, beispielsweise mit Lithiumisopropylamid in einem inerten organischen Lösungsmittel wie Tetrahydrofuran bei niedriger Temperatur, z. B. −78°C, erfolgen.

Das Lithiumenolat wird dann vorzugsweise in situ mit $MoO_5$ · py · HPMT zweckmäßig bei Temperaturen zwischen etwa Raumtemperatur und −78°C behandelt oder mit Sauerstoff in Gegenwart eines Trialkylphosphits, z. B. Triäthylphosphit, wobei zweckmäßig Sauerstoff durch ein Gemisch des Enolats und des Trialkylphosphits in einem inerten organischen Lösungsmittel wie Tetrahydrofuran bei niederer Temperatur, z. B. bei −78°C geleitet wird.

Die Verbindung der Formel XXXIII wird dann durch Ersatz der Alkylendioxygruppe durch eine Alkylendithiogruppe, insbesondere die Äthylendithiogruppe in einer Verbindung der Formel XVI übergeführt. Dieser Ersatz kann dadurch vorgenommen werden, daß man die Alkylendioxyverbindung mit einem geeigneten Alkandithiol, z. B. Äthandithiol in Gegenwart von Borfluoridätherat behandelt, zweckmäßig in einem inerten organischen Lösungsmittel wie einem halogenierten Kohlenwasserstoff, z. B. Dichlormethan, bei Temperaturen um 0°C. Die Ausgangsstoffe der Formel III sind bekannte Verbindungen oder Analoga von bekannten Verbindungen. Die Verbindungen der Formel I und deren pharmazeutisch anwendbaren Säureadditionssalze besitzen antibiotische und Antitumor-Aktivität.

Andere Anthracyclinglykoside gleicher Wirkungsrichtung, z. B. das 4-Demethoxydaunomycin und das 11-Deoxy-13-dihydrodaunomycin sind aus der FR-A-2 355 028 bzw. der FR-A-2 424 285 bekannt.

Die Aktivität der Verbindungen der Formel I und deren Salzen läßt sich mit standardisierten pharmakologischen Testverfahren zeigen. Beispielsweise kann die Antitumor-Aktivität in vivo mit folgendem Test gezeigt werden:

Als Versuchstier werden Laboratoriumsmäuse verwendet. Die Testsubstanzen werden in Wasser gelöst oder, falls unlöslich, in Propylenglykol suspendiert. Lösungen bzw. Suspensionen werden nur 2 Tage lang verwendet und im Dunkeln bei 4°C aufbewahrt. Die Mäuse erhielten Injektionen von $10^5$ lebensfähigen Lymphozytenleukämietumorzellen. Die Behandlung wurde am gleichen Tag mit 5täglichen Intraperitonealinjektionen der Testsubstanzen pro Woche begonnen. Nicht behandelte Kontrolltiere starben zwischen dem 9. und 12. Tag. Die Wirksamkeit der Behandlung wird ausgedrückt als der Quotient T/C, der die mittlere Überlebenszeit der behandelten Tiere geteilt durch die mittlere Lebenszeit der Kontrolltiere angibt. In diesem Test hatte das (1 S)-cis-1-[(3-Amino-2,3,6-trideoxy-$\alpha$-L-lyxohexopyranosyl)-oxy]-1,2,3,4,6,11-hexahydro-3,5,12-trihydroxy-3-hydroxymethyl-6,11-dioxonaphthacen einen T/C-Wert von 2,31 bei einer Dosierung von 0,1 mg/kg i. p. und das (1 S)-cis-1-[(3-Amino-2,3,6-trideoxy-$\alpha$-L-lyxohexopyranosyl)oxy]-1,2,3,4,6,11-hexahydro-3,5,12-trihydroxy-3-methyl-6,11-dioxonaphthacen einen T/C-Wert von 4,0 bei einer Dosierung von 0,5 mg/kg i. p.

Die Verbindungen der Formel I und deren pharmazeutisch anwendbare Säureadditionssalze können als Medikamente verwendet werden, beispielsweise in Form pharmazeutischer Präparate, die sie zusammen mit einem verträglichen Träger enthalten. Der Träger kann ein inertes organisches oder anorganisches Material sein, das für enterale, z. B. orale oder parenterale Verabreichung geeignet ist. Beispiele solcher Träger sind Wasser, Gelatine, Talk, Stärke, Magnesiumstearat, Gummi arabicum, vegetabile Öle, Polyalkylenglykole und Vaseline. Die pharmazeutischen Präparate können in herkömmlicher Weise hergestellt werden und in fester Form (z. B. Tabletten, Dragées, Suppositorien oder Kapseln) oder flüssiger Form (z. B. Lösungen, Suspensionen oder Emulsionen) vorliegen. Die pharmazeutischen Präparate können üblichen pharmazeutischen Operationen wie Sterilisation unterworfen werden und/oder können zusätzliche konventionelle Zusatzstoffe wie Konservierungsmittel, Stabilisatoren, Netzmittel, Puffer, Salze zur Variierung des osmotischen Druckes enthalten. Sie können auch noch andere therapeutisch wertvolle Stoffe enthalten.

Die Verbindungen der Formel I und deren pharmazeutisch anwendbare Salze können zur Behandlung von menschlichen malignen Tumoren fortlaufend (z. B. täglich) oder periodisch (z. B. monatlich) verabreicht werden. Im allgemeinen beträgt die Dosierung zwischen 25 $mg/m^2$ bis 700 $mg/m^2$ (Milligramm pro Quadratmeter Hautoberfläche). Diese Dosierung ist jedoch nur beispielhaft und kann nach unten oder oben in Abhängigkeit von Faktoren wie der besonderen zu verabreichenden Verbindungen, der Verabreichungsweise und der Schwere der Erkrankung nach Weisung des Arztes variiert werden.

Die folgenden Beispiele illustrieren die Erfindung weiter:

## Beispiel 1

(A) Eine Lösung von 1 g (1 S)-cis-3-Acetoxymethyl-1,2,3,4,6,11-hexahydro-1,3,5,12-tetrahydroxy-6,11-dioxonapthacen in 100 ml Tetrahydrofuran wurde bei −5° mit einem 1 g 2,3,6-Trideoxy-4-O-p-nitrobenzoyl-3-trifluoracetamido-$\alpha$-L-lyxopyranosylchlorid versetzt. Das Gemisch wurde unter Rühren im Verlaufe von 20 Minuten mit einer Lösung von 0,48 g Silbertrifluormethansulfonat in 15 ml trockenem Äther versetzt. Danach wurden weitere 1 g des oben erwähnten Chlorzuckers zugesetzt und weitere 0,48 g Silbertrifluormethansulfonat in 15 ml trockenem Äther im Verlauf von 20 Minuten. Das Gemisch wurde dann eine halbe Stunde bei −5° gerührt, dann in 300 ml 10%ige Kaliumhydrogencarbonatlösung gegossen und mit viermal 100 ml Dichlormethan extrahiert. Die Dichlormethanextrakte

wurden über Natriumsulfat getrocknet und eingedampft und lieferten einen roten Gummi, der durch Säulenchromatographie an Silicagel mit Hexan/Äthylacetat (1 : 1 Volumenteile) gereinigt wurde. Neben 132 mg nicht umgesetztem Dioxonaphthacen-Ausgangsmaterial wurden 1,4 g (1S)-cis-3-Acetoxy-methyl-1-[(2,3-2,6-trideoxy-3-trifluoracetamido-4-O-p-nitrobenzoyl-$\alpha$-L-lyxohexopyranosyl)oxy]-1,2,3,4,6,11-hexahydro-3,5,12-trihydroxy-6,11-dioxonaphthacen als roter Gummi erhalten, der ohne weitere Reinigung verwendet wurde.

(B) 1,4 g der so erhaltenen Verbindung wurden in einem Gemisch von 40 ml Dichlormethan und 100 ml Methanol gelöst. Die Lösung wurde auf 0°C gekühlt und tropfenweise mit 0,1 M wäßriger Natriumhydroxidlösung versetzt, bis eine dauernde Braun-Purpur-Färbung auftrat. Nach 10 Minuten zeigte die Dünnschichtchromatographie, daß kein Ausgangsmaterial mehr vorhanden war. Die Reaktion wurde dann durch Zusatz von Essigsäure bis zum Auftreten einer orangeroten Färbung abgebrochen, das Gemisch mit 250 ml Wasser verdünnt und mit viermal 100 ml Dichlormethan extrahiert. Die Extrakte wurden über Natriumsulfat getrocknet und eingedampft und lieferten einen orangen Gummi, der durch Säulenchromatographie mit Aceton/Dichlormethan (1 : 10 Volumenteile) als Elutionsmittel gereinigt wurde. Kristallisation aus Aceton/Diäthyläther lieferte 0,9 g (1S)-cis-3-Acetoxy-methyl-1-[(2,3,6-trideoxy-3-trifluoracetamido-$\beta$-L-lyxohexopyranosyl)oxy]-1,2,3,4,6,11-hexahydro-3,5,12-trihydroxy-6,11-dioxonaphthacen in Form orange-roter Kristalle vom Schmelzpunkt 138—141°; $[\alpha]_D^{29}$ = +170,3° (c = 0,1 % in Chloroform).

(C) 0,8 g dieser Verbindung wurde in einem Gemisch von 100 ml Dichlormethan und 50 ml Methanol gelöst. Die Lösung wurde auf 0° gekühlt und mit 0,1 M wäßriger Natronlauge bis zum Auftreten einer tief purpurnen Färbung versetzt. Die Lösung wurde auf Raumtemperatur aufwärmen gelassen und 2—2½ Stunden gerührt, bis die Dünnschichtchromatographie zeigte, daß kein Ausgangsmaterial mehr vorhanden war. Die Reaktion wurde dann durch Zusatz von Essigsäure bis zum Auftreten einer orangeroten Farbe abgebrochen, die Lösung mit 250 ml Wasser verdünnt und mit viermal 100 ml Dichlormethan extrahiert. Die Extrakte wurden über Natriumsulfat getrocknet und eingedampft, wobei ein oranger Feststoff erhalten wurde. Krsitallisation aus Tetrahydrofuran/Diäthyläther lieferte 0,65 g (1S)-cis-1-[(2,3,6-Trideoxy-3-trifluoracetamido-$\alpha$-L-lyxohexapyranosyl)oxy]-1,2,3,4,6,11-hexahydro-3,5,12-trihydroxy-3-hydroxymethyl-6,11-dioxonaphthacen als orangerote Kristalle, Schmelzpunkt 239—240°; $[\alpha]_D^{20}$ = +151,6° (c = 0,1 % in Chloroform).

(D) 500 mg der so erhaltenen Verbindung wurden in 50 ml 0,1 M wäßriger Natriumhydroxidlösung gelöst und bei Raumtemperatur 45 Minuten gerührt. Die Lösung wurde durch Zusatz von 0,1 M Salzsäure auf pH 8—9 eingestellt und dann mehrmals mit Dichlormethan, das 10 % Äthanol enthielt, extrahiert, bis die Extrakte vollständig farblos waren. Die vereinigten Extrakte wurden mit Wasser gewaschen, über Natriumsulfat getrocknet und zu einem roten Feststoff eingedampft. Der Feststoff wurde in 10 ml Dichlormethan, das 2 ml Methanol enthielt, gelöst und filtriert. Danach wurden unter Umschwenken 4 ml 0,25 M methanolische Salzsäure zugesetzt und die Lösung auf ca. 5 ml eingeengt. Nach Ausfällung durch Zusatz von 50 ml wasserfreiem Diäthyläther, Filtrieren und Waschen des Filterrückstandes mit Diäthyläther und Trocknen unter vermindertem Druck erhielt man 455 mg (1S)-cis-1-[(3-Amino-2,3,6-trideoxy-$\alpha$-L-lyxohexopyranosyl)oxy]-1,2,3,4,6,11-hexahydro-3,5,12-trihydroxy-3-hydroxymethyl-6,11-dioxonaphthacen-hydrochlorid als orange-roten Feststoff vom Schmelzpunkt 183—186° (Zersetzung); $[\alpha]_D^{20}$ = +153,2° (c = 0,05 % in Methanol).

Das als Ausgangsmaterial verwendete (1S)-cis-3-Acetoxymethyl-1,2,3,4,6,11-hexahydro-1,3,5,12-tetrahydroxy-6,11-dioxonaphthacen kann wie folgt hergestellt werden:

(a) Eine Lösung von 2 mMol (1S)-cis-3-Acetoxymethyl-1,2,3,4,5,12-hexahydro-5,12-dioxonaphthacen-1,3-diol und 244 mg (2 mMol) Benzolboronsäure in einem Gemisch von 150 ml Benzol und 0,5 ml Eisessig wurde unter Rühren 1 Stunde zum Rückfluß erhitzt. Das Gemisch wurde abkühlen gelassen und das Lösungsmittel abgedampft, wobei ein gelber Rückstand erhalten wurde. Der Rückstand wurde mit 50 ml Diäthyläther verrieben und filtriert und lieferte (1S)-cis-3-Acetoxymethyl-1,2,3,4,5,12-hexahydro-5,12-dioxo-1,3-naphthacendiyl-benzolboronat in Form eines gelben Pulvers, das ohne weitere Reinigung verwendet wurde.

(b) 1,78 mMol des vorstehend erhaltenen Benzolboronats wurde in einem Gemisch von 40 ml trockenem Pyridin und 20 ml Acetanhydrid gelöst. Nach Zusatz von 10% Palladium auf Kohle-Katalysator wurde das Gemisch bei Raumtemperatur und Atmosphärendruck eine halbe Stunde lang hydriert. Der Katalysator wurde abfiltriert und das Filtrat mit 160 ml Dichlormethan verdünnt. Die erhaltene Lösung wurde mit dreimal 200 ml Wasser gewaschen, über Natriumsulfat getrocknet, filtriert und eingedampft. Der Rückstand wurde mit Diäthyläther verrieben und filtriert und lieferte 99 % (1S)-cis-5,12-Diacetoxy-3-acetoxymethyl-1,2,3,4-tetrahydro-1,3-naphthacendiyl-benzolboronat in Form hellgelber Kristalle, Schmelzpunkt 256—258°; $[\alpha]_D^{20}$ = +251,3° (c = 0,1 % in Dioxan).

(c) 0,6 mMol des gemäß Abschnitt (b) Benzolboronats wurden in einem Gemisch von 18 ml Eisessig und 6 ml Acetanhydrid gelöst. Nach Zusatz von 140 mg (2,4 mMol) fein gemahlenem Chromtrioxid wurde das Gemisch bei Raumtemperatur 16 Stunden gerührt. Das Gemisch wurde dann in 250 ml Wasser gegossen und die erhaltene Suspension mit zweimal 200 ml Dichlormethan extrahiert. Die vereinigten Dichlormethanextrakte wurden eingedampft und der Rückstand mit Diäthyläther verrieben und filtriert und lieferte (1S)-cis-5,12-Diacetoxy-3-acetoxymethyl-1,2,3,4,6,11-hexahydro-6,11-dioxo-1,3-naphthacendiyl-benzolboronat in Form hellgelber Kristalle vom Schmelzpunkt 204—205°;

$[\alpha]_D^{20} = +180,3°$ (c = 0,1 % in Dioxan).

(d) Eine Lösung von 0,17 mMol des gemäß Absatz (c) erhaltenen Benzolboronats in 20 ml Dichlormethan wurde unter Rühren auf −78° gekühlt. Danach wurde eine Lösung von 125 mg Bortrichlorid in 5 ml Dichlormethan zugesetzt und das Gemisch wurde unter Rühren im Verlauf 1 Stunde auf −10° aufwärmen gelassen. Das Gemisch wurde dann in 20 ml eiskalte 2 M Salzsäure gegossen. Nach Trennung der Schichten wurde die organische Phase mit 20 ml Wasser gewaschen, über Natriumsulfat getrocknet, filtriert und eingedampft. Der Rückstand wurde mit 5 ml Diäthyläther verrieben und filtriert und lieferte (1 S)-cis-3-Acetoxymethyl-1,2,3,4,6,11-hexahydro-5,12-dihydroxy-6,11-dioxo-1,3-naphthacendiyl-benzolboronat in Form eines roten halbfesten Stoffes, der ohne weitere Reinigung verwendet wurde.

(e) 0,10 mMol des gemäß Absatz (d) erhaltenen Benzolboronats wurden in 6 ml Dichlormethan gelöst. Nach Zusatz von 1,5 ml 2-Methyl-2,4-pentandiol und 0,25 ml Eisessig wurde die Lösung bei Raumtemperatur 40 Stunden gerührt. Die Lösung wurde dann mit dreimal 15 ml Wasser gewaschen, getrocknet, filtriert und eingedampft. Der ölig-kristalline Rückstand wurde mit Diäthyläther verrieben und filtriert und lieferte (1 S)-3-Acetoxymethyl-1,2,3,4,6,11-hexahydro-1,3,5,12-tetrahydroxy-6,11-dioxonaphthacen, das nach Reinigung durch Chromatographie an Silicagel mit 5 % Methanol in Toluol rote Kristalle vom Schmelzpunkt 201—203° lieferte; $[\alpha]_D^{20} = +119,8°$ (c = 0,1 % in Dioxan).

Das im Abschnitt (a) dieses Beispiels als Ausgangsmaterial verwendete (1 S)-cis-3-Acetoxymethyl-1,2,3,4,5,12-hexahydro-5,12-dioxonaphthacen-1,3-diol kann wie folgt hergestellt werden:

(i) 40 g rac-1,2,3,4-Tetrahydro-5,8-dimethoxy-4-oxonaphthalin-2-carbonsäure-methylester wurden zu einem Gemisch von 800 ml Toluol, 800 ml Hexan, 30 ml Äthylenglykol und 0,65 g Toluol-4-sulfonsäure gegeben. Das Gemisch wurde 24 Stunden zum Rückfluß erhitzt unter Verwendung eines Wasserabscheiders. Die Lösung wurde in ein Eisbad gekühlt und mit dreimal 124 ml 10 %iger Kaliumhydrogencarbonatlösung und 200 ml Kochsalzlösung gewaschen, getrocknet und eingedampft. Der Rückstand wurde in 200 ml Methanol bei 70° aufgenommen und die Lösung mit 0,5 g einer 50 %igen Dispersion von Natriumhydrid in Mineralöl versetzt. Die Lösung wurde auf Zimmertemperatur und dann 2 Stunden in einem Eisbad gekühlt. Das Kristallisat wurde abfiltriert, mit kaltem Methanol gewaschen und unter vermindertem Druck getrocknet. Man erhielt 28,5 g rac-4,4-Äthylendioxy-1,2,3,4-tetrahydro-5,8-dimethoxynaphthalin-2-carbonsäure-methylester als farblose Kristalle vom Schmelzpunkt 133—134°.

(ii) (a) Zu einer Lösung von 84 ml Diisopropylamin in 250 ml trockenem Tetrahydrofuran wurden bei −78° unter Argonatmosphäre 39 ml einer 1,6 molaren Lösung von n-Butyllithium in Hexan gegeben. Das Gemisch wurde 10 Minuten gerührt und dann mit einer Lösung von 12,32 g 4,4-Äthylendioxy-1,2,3,4-tetrahydro-5,8-dimethoxynaphthalin-2-carbonsäure-methylester in 75 ml trockenem Tetrahydrofuran rasch versetzt. Das Gemisch wurde 50 Minuten unter Rühren bei −78° gehalten und dann mit 27,8 g fein gemahlenem $MoO_5py \cdot HPMT$ versetzt. Nach weiteren 80 Minuten wurde das Gemisch auf 0° aufgewärmt und 20 Minuten gerührt, worauf es mit 400 ml Wasser versetzt wurde. Nach 10 Minuten wurde das Tetrahydrofuran größtenteils unter vermindertem Druck verdampft und der wäßrige Rückstand mit fünfmal 200 ml Äthylacetat extrahiert. Die Extrakte wurden über Magnesiumsulfat getrocknet, filtriert und eingedampft und lieferten ein Öl, das durch Chromatographie an Silicagel mit Äthylacetat/Hexan (1 : 1 Volumenteile) gereinigt wurde. Nach Elution von 1,57 g Ausgangsmaterial erhielt man 7,51 g rac-4,4-Äthylendioxy-1,2,3,4-tetrahydro-2-hydroxy-5,8-dimethoxynaphthalin-2-carbonsäure-methylester als farblose Kristalle vom Schmelzpunkt 74—75°.

(ii) (b) Aus 9,84 g rac-4,4-Äthylendioxy-1,2,3,4-tetrahydro-5,8-dimethoxynaphthalin-2-carbonsäure-methylester wurde wie in (ii) (a) beschrieben, das Lithiumenolat von rac-4,4-Äthylendioxy-1,2,3,4-tetrahydro-5,8-dimethoxynaphthalin-2-carbonsäure-methylester hergestellt. Das Enolat wurde im Verlaufe von 5 Minuten bei −78° zu einer gerührten Lösung von 11,2 ml trockenem Triäthylphosphit in 60 ml Tetrahydrofuran durch das ein rascher Sauerstoffstrom geleitet wurde, gegeben. Die Sauerstoffzufuhr wurde 50 Minuten fortgesetzt und die Temperatur bei −78° gehalten. Die Reaktion wurde dann durch Zusatz von 8,8 ml Essigsäure beendet. Das Kühlbad wurde entfernt und nach 5 Minuten wurden 200 ml Wasser zugegeben. Nach weiteren 20 Minuten wurde der größte Teil des Tetrahydrofurans abgedampft und das Produkt mit viermal 100 ml Äthylacetat aufgenommen. Die Äthylacetatextrakte wurden mit 200 ml 10%iger wäßriger Kaliumhydrogencarbonatlösung gewaschen, über Magnesiumsulfat getrocknet, filtriert und eingedampft und lieferten ein gelbes Öl, das in 130 ml Äther gelöst und kristallisiert wurde. Man erhielt 6,86 g rac-4,4-Äthylendioxy-1,2,3,4-tetrahydro-2-hydroxy-5,8-dimethoxynaphthalin-2-carbonsäure-methylester in Form farbloser Kristalle vom Schmelzpunkt 74—75°.

(iii) 10 g rac-4,4-Äthylendioxy-1,2,3,4-tetrahydro-2-hydroxy-5,8-dimethoxynaphthalin-2-carbonsäure-methylester wurden in 30 ml Dichlormethan gelöst. Die Lösung wurde bei 0° mit 4 ml Äthandithiol und anschließend mit 4 ml Bortrifluoridätherat versetzt. Das Gemisch wurde 15 Minuten bei 0° gerührt und dann in 200 ml Diäthyläther gegossen. Die organische Phase wurde mit dreimal 50 ml 5 %iger Natriumhydroxidlösung gewaschen und eingedampft, wobei ein gelbes Öl erhalten wurde, das in 200 ml Methanol aufgenommen wurde. Die Lösung wurde mit 10 ml 5 %iger Natronlauge versetzt und 1,5 Stunden bei Raumtemperatur gerührt. Der Hauptteil des Methanols wurde dann abgedampft und der Rückstand mit 250 ml Wasser verdünnt und mit dreimal 100 ml Äther extrahiert. Die wäßrige

Phase wurde mit Salzsäure angesäuert und das ausgeschiedene Öl erstarren gelassen. Das Produkt wurde durch Filtration gesammelt, mit Wasser säurefrei gewaschen und getrocknet. Das Rohprodukt wurde in 150 ml Äthylacetat suspendiert und 30 Minuten zum Rückfluß erhitzt. Das Gemisch wurde gekühlt und das Produkt nach 24 Stunden abfiltriert. Man erhielt 7,0 g 1',2',3',4'-Tetrahydro-2'-hydroxy-5',8'-dimethoxyspiro[1,3-dithiolan-2,4'-naphthalin]-2'-carbonsäure in Form farbloser Kristalle vom Schmelzpunkt 189—189,5°.

(iv) Eine Suspension von 3,42 g der vorstehend erhaltenen Spiroverbindung in 168 ml Äthylacetat wurde mit 4,0 g Brucin zum Rückfluß erhitzt, bis eine klare Lösung entstanden war. Nach Animpfen ließ man die Lösung langsam auf Raumtemperatur abkühlen. Der kristalline Niederschlag (3,6 g) wurde nach 2 Tagen abgetrennt. Er wurde in 1500 ml siedendem Äthylacetat gelöst, die Lösung auf 600 ml konzentriert und langsam abkühlen gelassen. Das kristalline Produkt (2,7 g $[\alpha]_D^{20} = -46,6°$, c = 0,5 % in Dimethylformamid) wurde in 150 ml Äthylacetat suspendiert und mit dreimal 10 ml 5 N Salzsäure und zweimal 100 ml Kochsalzlösung ausgeschüttelt. Nach Trocknen über Magnesiumsulfat wurde das Lösungsmittel abgedampft und man erhielt ein farbloses Öl, das aus Diäthyläther kristallisiert 1,22 g (R)-1',2',3',4'-Tetrahydro-2'-hydroxy-5',8'-dimethoxyspiro[1,3-dithiolan-2,4'-naphthalin]-2'-carbonsäure in Form farbloser Kristalle vom Schmelzpunkt 147—149°; $[\alpha]_D^{20} = +13,8°$ (c = 0,5 % in Dioxan) lieferte.

(v) Die Äthylacetat-Mutterlaugen der ersten Kristallisation des im vorhergehenden Abschnitt beschriebenen Verfahren wurden mit dreimal 10 ml 5 N Salzsäure und zweimal 100 ml Kochsalzlösung ausgeschüttelt, getrocknet und eingedampft und lieferten 1,7 g eines Feststoffes. Dieser wurde in 50 ml Äthylacetat suspendiert und eine halbe Stunde zum Rückfluß erhitzt. Nach Abkühlung wurden 0,6 g rac-1',2',3',4'-Tetrahydro-2'-hydroxy-5',8'-dimethoxyspiro[1,3-dithiolan-2,4'-naphthalin]-2'-carbonsäure vom Schmelzpunkt 189—190° erhalten. Die Mutterlaugen wurden eingedampft und der Rückstand in Diäthyläther aufgenommen, filtriert und kristallisiert. Man erhielt 1,12 g (S)-1',2',3',4'-Tetrahydro-2'-hydroxy-5',8'-dimethoxyspiro[1,3-dithiolan-2,4'-naphthalin]-2'-carbonsäure in Form farbloser Kristalle vom Schmelzpunkt 145—148°; $[\alpha]_D^{20} = -13,5°$ (c = 0,5 % in Dioxan).

(vi) 20 g (S)-1',2',3',4'-Tetrahydro-2'-hydroxy-5',8'-dimethoxyspiro[1,3-dithiolan-2,4'-naphthalin]-2'-carbonsäure wurden in 200 ml Methanol suspendiert und mit 40 ml Bortrifluorid/Methanol versetzt. Das Gemisch wurde 3,5 Stunden bei Raumtemperatur gerührt bis sich eine klare Lösung ergab. Nach Abdampfen von etwa 80 ml Methanol wurde die Lösung in 400 ml Dichlormethan gegossen. Die organische Lösung wurde mit 500 ml Wasser, 200 ml 10%iger Kaliumhydrogencarbonatlösung und 200 ml Kochsalzlösung gewaschen. Nach Trocknen über Magnesiumsulfat wurde das Lösungsmittel abgedampft und man erhielt 24 g eines gelben Gummis. Kristallisation dieses Produkts aus Diäthyläther/Hexan lieferte 19,5 g (S)-1',2',3',4'-Tetrahydro-2'-hydroxy-5',8'-dimethoxyspiro[1,3-dithiolan-2,4' - naphthalin] - 2' - carbonsäure - methylester in Form farbloser Kristalle vom Schmelzpunkt 115—117°; $[\alpha]_D^{20} = -12,7°$ (c = 0,5 % in Chloroform).

(vii) 2 g dieses Methylesters wurden in 200 ml trockenem Tetrahydrofuran gelöst und die Lösung mit 2,0 g Natriumborhydrid versetzt. Das Gemisch wurde 20 Stunden unter Stickstoff bei Raumtemperatur gerührt, das Lösungsmittel abgedampft und 100 ml 10%ige Ammoniumchloridlösung zugesetzt. Das Gemisch wurde mit dreimal 30 ml Äthylacetat extrahiert, die Extrakte getrocknet und eingedampft und lieferten (S)-1',2',3',4'-Tetrahydro-3'-hydroxy-3'-hydroxymethyl-5',8'-dimethoxyspiro[1,3-dithiolan-2,1'-naphthalin] in Form eines farblosen Gummis, der direkt im nächsten Schritt weiter verwendet wurde.

(viii) 1,6 g der Hydroxymethylverbindung wurden in 30 ml trockenem Pyridin gelöst und die Lösung mit 1,5 g Acetanhydrid versetzt. Das Gemisch wurde 20 Stunden bei Raumtemperatur stehen gelassen und dann in eiskalte 5 M Schwefelsäure gegossen. Das erhaltene Gemisch wurde mit Äthylacetat extrahiert, die Extrakte mit Wasser und Natriumhydrogencarbonatlösung gewaschen, getrocknet und eingedampft. Man erhielt (S)-3'-Acetoxymethyl-1',2',3',4'-tetrahydro-3'-hydroxy-5',8'-dimethoxyspiro-[1,3-dithiolan-2,1'-naphthalin] in Form eines farblosen Öls, das direkt in der nächsten Stufe verwendet wurde.

(ix) 1,9 g der oben erhaltenen Acetoxymethylverbindung in 40 ml Tetrahydrofuran wurden zu einer gerührten Suspension von 6,4 g Quecksilber(II)chlorid und 6,4 g Quecksilber(II)oxid in 200 ml Methanol, die 18 ml Wasser enthielten, gegeben. Nach 1stündigem Stehen der Raumtemperatur wurden etwa 150 ml Lösungsmittel unter vermindertem Druck abgedampft, dann wurden 200 ml Dichlormethan zugegeben und die erhaltene Suspension wurde vom Unlöslichen abfiltriert. Das Filtrat wurde mit dreimal 200 ml Wasser gewaschen, über Magnesiumsulfat getrocknet und eingedampft. Der erhaltene feste Rückstand wurde mit Diäthyläther verrieben und lieferte (S)-3-Acetoxymethyl-1,2,3,4-tetrahydro-3-hydroxy-5,8-dimethoxy-1-oxo-naphthalin in Form eines grauweißen Pulvers, das für die nächste Stufe ohne weitere Reinigung weiter verwendet wurde.

(x) 2,37 g des vorstehend erwähnten Ketons wurden in 100 ml Tetrahydrofuran gelöst und die Lösung mit 2,0 g Natriumborhydrid versetzt. Nach 2 Stunden Rühren bei Raumtemperatur wurde das Lösungsmittel abgedampft und 100 ml 10%iger Ammonchloridlösung wurden zugesetzt. Das Gemisch wurde mit dreimal 100 ml Äthylacetat extrahiert, die Extrakte getrocknet und eingedampft. Der erhaltene farblose Gummi wurde in 200 ml Äthylacetat gelöst und mit 2,0 g Benzolboronsäure und 3 Tropfen Essigsäure versetzt. Das Gemisch wurde 1 Stunde zum Rückfluß erhitzt, das Lösungsmittel eingedampft und

der Rückstand mit 200 ml Toluol versetzt. Nach Zusatz von 75 mg Toluol-4-sulfonsäure wurde die Lösung 4,5 Stunden bei Raumtemperatur gerührt, mit 50 ml 10%iger Kaliumhydrogencarbonatlösung gewaschen, getrocknet und eingedampft. Man erhielt rohes (S)-3-Acetoxymethyl-1,2,3,4-tetrahydro-5,8-dimethoxy-naphthalin-1,3-diyl-benzolboronat in Form eines halbfesten Rückstandes. Dieser Rückstand wurde in Dichlormethan, das etwas Essigsäure enthielt, gelöst. Die Lösung wurde mit 2-Methyl-2,4-pentandiol versetzt und 24 Stunden bei Raumtemperatur stehen gelassen. Das Gemisch wurde in 5%ige Kaliumhydrogencarbonatlösung gegossen und dreimal mit Dichlormethan extrahiert. Die Extrakte wurden über Magnesiumsulfat getrocknet und eingedampft. Das erhaltene farblose Öl wurde in Hexan gelöst und bei 4° über Nacht Kristallisieren gelassen. Die Filtration lieferte (S)-cis-3-Acetoxymethyl-1,2,3,4-tetrahydro-5,8-dimethoxynaphthalin-1,3-diol in Form farbloser Kristalle vom Schmelzpunkt 97—98°; $[\alpha]_D^{20}$ = −2,6° (c = 0,5% in Chloroform).

(xi) Eine Lösung von 1,1 g Ammoniumcernitrat in 20 ml Wasser wurde unter Rühren zu einer Lösung von 296 mg des vorstehend genannten Diols in 20 ml Acetonitril gegeben. Nach 5 Minuten Rühren bei Raumtemperatur wurde das Gemisch in 200 ml Wasser gegossen und mit sechsmal 50 ml Dichlormethan extrahiert. Die vereinigten Extrakte wurden über Magnesiumsulfat getrocknet und eingedampft und lieferten (S)-cis-3-Acetoxymethyl-1,2,3,4-5,8-hexahydro-5,8-dioxo-naphthalin-1,3-diol, das in 20 ml Xylol gelöst wurde.

(xii) Die so erhaltene Lösung wurde mit 0,3 g trans-1,2-Diacetoxy-1,2-dihydrobenzocyclobuten versetzt und das Gemisch 2 Stunden auf 140° erhitzt. Nach Abkühlen wurde die Lösung über Silicagel filtriert und das Lösungsmittel eingedampft, wobei ein hellgelber Rückstand erhalten wurde. Verreiben des Rückstandes mit Äthylacetat/Diäthyläther lieferte 220 mg (1 S)-cis-3-Acetoxymethyl-1,2,3,4,5,-12-hexahydro-5,12-dioxonaphthacen-1,3-diol in Form gelber Kristalle vom Schmelzpunkt 189—191°; $[\alpha]_D^{20}$ = +40,3° (c = 0,5% in Chloroform).

Beispiel 2

(A) In Analogie zu Beispiel 1 (A) wurde aus (1 S)-cis-1,2,3,4,6,11-Hexahydro-1,3,5,12-tetrahydroxy-3-methyl-6,11-dioxonaphthacen nach Kristallisation aus Tetrahydrofuran/Hexan das (1 S)-cis-1-[(2,3,6-Trideoxy-3-trifluoracetamido-4-O-p-nitrobenzoyl-α-L-lyxohexopyranosyl)oxy]-1,2,3,4,6,11-hexahydro-3,5,12-trihydroxy-3-methyl-6,11-dioxonaphthacen in Form orange-roter Kristalle vom Schmelzpunkt 225—226°; $[\alpha]_D^{20}$ = −101,8° (c = 0,1% in Chloroform) erhalten.

(B) Das so erhaltene Produkt wurde wie in Beispiel 1 (B) behandelt und lieferte (1 S)-cis-1-[(2,3,6-Trideoxy-3-trifluoracetamido-α-L-lyxohexopyranosyl)oxy]-1,2,3,4,6,11-hexahydro-3,5,12-trihydroxy-3-methyl-6,11-dioxonaphthacen in Form orange-roter Kristalle vom Schmelzpunkt 253—254°; $[\alpha]_D^{20}$ = +180,9° (c = 0,1% in Chloroform).

(C) Das gemäß Abschnitt (B) erhaltene Produkt wurde gemäß dem Verfahren von Beispiel 1 (D) behandelt und lieferte (1 S)-cis-1-[(3-Amino-2,3,6-trideoxy-α-L-lyxohexopyranosyl)-oxy]-1,2,3,4,6,11-hexahydro-3,5,12-trihydroxy-3-methyl-6,11-dioxonaphthacen-hydrochlorid in Form orange-roter Kristalle vom Schmelzpunkt 174—176° (Zersetzung); $[\alpha]_D^{20}$ = +160,1° (c = 0,05% in Methanol).

Das als Ausgangsmaterial verwendete (1 S)-cis-1,2,3,4,6,11-Hexahydro-1,3,5,12-tetrahydroxy-3-methyl-6,11-dioxonaphthacen kann wie folgt hergestellt werden:

(a) In Analogie zu Beispiel 1 (a) wurde aus (1 S)-cis-1,2,3,4,5,12-hexahydro-3-methyl-5,12-dioxonaphthacen-1,3-diol das (1 S)-cis-1,2,3,4,5,12-Hexahydro-3-methyl-5,12-dioxo-1,3-naphthacendiyl-benzolboronat erhalten, das im nächsten Schritt ohne weitere Reinigung verwendet wurde.

(b) In Analogie zu Beispiel 1 (b) wurde aus (1 S)-cis-1,2,3,4,5,12-Hexahydro-3-methyl-5,12-dioxo-1,3-naphthacen-diyl-benzolboronat das (1 S)-cis-5,12-Diacetoxy-1,2,3,4-tetrahydro-3-methyl-1,3-naphthacendiyl-benzolboronat erhalten, das ohne weitere Reinigung im nächsten Schritt verwendet wurde.

(c) In Analogie zu Beispiel 1 (c) wurde (1 S)-cis-5,12-Diacetoxy-1,2,3,4-tetrahydro-3-methyl-1,3-naphthacendiyl-benzolboronat zu (1 S)-cis-5,12-Diacetoxy-1,2,3,4,6,11-hexahydro-3-methyl-6,11-dioxo-1,3-naphthacendiyl-benzolboronat oxidiert. Das Produkt wurde im nächsten Schritt ohne Reinigung eingesetzt.

(d) In Analogie zu Beispiel 1 (d) wurde aus (1 S)-cis-5,12-Diacetoxy-1,2,3,4,6,11-hexahydro-3-methyl-6,11-dioxo-1,3-naphthacendiyl-benzolboronat mit Bortrichlorid das (1 S)-cis-1,2,3,4,6,11-Hexahydro-5,12-dihydroxy-3-methyl-6,11-dioxo-1,3-naphthacendiyl-benzolboronat erhalten, das im nächsten Schritt ohne Reinigung weiter verwendet wurde.

(e) In Analogie zu Beispiel 1 (e) wurde aus (1 S)-cis-1,2,3,4,6,11-Hexahydro-5,12-dihydroxy-3-methyl-6,11-dioxo-1,3-naphthacendiyl-benzolboronat das (1 S)-cis-1,2,3,4,6,11-Hexahydro-1,3,5,12-tetrahydroxy-3-methyl-6,11-dioxonaphthacen erhalten. Reinigung des Produktes durch Säulenchromatographie an Silicagel mit 5% Methanol in Toluol als Elutionsmittel lieferte rote Kristalle vom Schmelzpunkt 214—215°; $[\alpha]_D^{20}$ = +152,5° (c = 0,1% in Dioxan).

Das als Ausgangsmaterial im Abschnitt (a) dieses Beispiels verwendete (1 S)-cis-1,2,3,4,5,12-Hexahydro-3-methyl-5,12-dioxonapthacen-1,3-diol kann wie folgt hergestellt werden:

(i) 326 mg (S)-1',2',3',4'-Tetrahydro-3'-hydroxy-3'-hydroxymethyl-5',8'-dimethoxyspiro[1,3-dithio-

lan-2,1'-naphthalin], hergestellt gemäß Beispiel 1 (vii), wurden in 10 ml Pyridin gelöst. Die Lösung wurde bei 0° mit 400 mg Toluol-4-sulfonylchlorid versetzt und 20 Stunden bei 4° gehalten. Die Lösung wurde dann auf gestoßenes Eis gegossen, mit 5 N Schwefelsäure angesäuert und mit Äthylacetat extrahiert. Der Extrakt wurde mit Wasser und dann mit 5 %iger Kaliumhydrogencarbonatlösung gewaschen. Nach Trocknen und Abdampfen des Lösungsmittels wurde ein weißer Feststoff erhalten, der nach Verreiben mit Diäthyläther (S)-1',2',3',4'-Tetrahydro-3'-hydroxy-5',8'-dimethoxyspiro[1,3-dithiolan-2,1'-naphthyl]-3'-methyl-p-toluolsulfonat in Form farbloser Kristalle vom Schmelzpunkt 115° (Zersetzung) lieferte; $[\alpha]_D^{20} = -37,5°$ (c = 0,5 % in Chloroform).

(ii) 200 mg (S)-1',2',3',4'-Tetrahydro-3'-hydroxy-5',8'-dimethoxyspiro[1,3-dithiolan-2,1'-naphthyl]-3'-methyl-p-toluolsulfonat wurden in 20 ml trockenem Tetrahydrofuran, das 100 mg Lithiumaluminiumhydrid enthielt, gelöst. Das Gemisch wurde unter Stickstoff 3,5 Stunden zum Rückfluß erhitzt. Die Lösung wurde abgekühlt und die Reaktion durch Zusatz von gesättigter Ammoniumchloridlösung beendet. Das Lösungsmittel wurde abgedampft und der Rückstand in verdünnter Salzsäure aufgenommen. Die Lösung wurde mit Äthylacetat extrahiert, die Extrakte mit Wasser gewaschen, getrocknet und eingedampft. Das erhaltene farblose Öl wurde aus Diäthyläther kristallisiert und lieferte (S)-1',2',3',4'-Tetrahydro-3'-hydroxy-3'-methyl-5',8'-dimethoxyspiro[1,3-dithiolan-2,1'-naphthalin] in Form farbloser Kristalle vom Schmelzpunkt 152—153°; $[\alpha]_D^{20} = -48,0°$ (c = 0,5 % in Chloroform).

(iii) Die vorstehend erhaltene Verbindung wurde nacheinander gemäß den Verfahren der Beispiele 1 (ix) und (x) behandelt, ohne die Produkte zwischendurch zu reinigen. Man erhielt (S)-cis-1,2,3,4-Tetrahydro-3-methyl-5,8-dimethoxynaphthalin-1,3-diol in Form farbloser Kristalle vom Schmelzpunkt 166—167°; $[\alpha]_D^{20} = -7,8°$ (c = 0,5 % in Chloroform).

(iv) Das vorstehend erhaltene Diol wurde wie in den Beispielen 1 (xi) und 1 (xii) behandelt und lieferte (1 S)-cis-1,2,3,4,5,12-Hexahydro-3-methyl-5,12-dioxo-naphthacen-1,3-diol in Form gelber Kristalle vom Schmelzpunkt 209—211°; $[\alpha]_D^{20} = +52,6°$ (c = 0,5 % in Chloroform).

## Beispiel 3

(A) 1 g (1 S)-cis-1,2,3,4,6,11-Hexahydro-1,3,5,12-tetrahydroxy-3-methyl-6,11-dioxonaphthacen (hergestellt gemäß Beispiel 2 (e)) wurde in 100 ml Tetrahydrofuran gelöst. Die Lösung wurde bei −5° mit einer Lösung von 1 g 2,3,6-Trideoxy-4-O-p-nitrobenzoyl-3-trifluoracetamido-α-L-arabinohexopyranosylchlorid in 30 ml Dichlormethan versetzt. Das Gemisch wurde unter Rühren im Verlauf von 20 Minuten mit einer Lösung von 0,48 g Silberfluormethansulfonat in 15 ml trockenem Diäthyläther versetzt. Danach wurden weitere 0,5 g des genannten Chlorzuckers und anschließend weitere 0,24 g Silbertrifluormethansulfonat in 7,5 ml trockenem Diäthyläther zugegeben. Das Gemisch wurde eine halbe Stunde bei −5° gerührt, dann in 300 ml 10 %ige Kaliumhydrogencarbonatlösung gegossen und mit viermal 100 ml Dichlormethan extrahiert. Die Extrakte wurden über Natriumsulfat getrocknet und eingedampft und lieferten einen roten Gummi, der durch Säulenchromatographie an Silicagel mit Hexan/Äthylacetat (1 : 1 Volumenteile) gereinigt wurde. Neben 120 mg nicht umgesetztem Dioxonaphthacen-Ausgangsmaterial wurden 1,05 g (1 S)-cis-1-[(2,3,6-Trideoxy-3-trifluoracetamido-4-O-p-nitrobenzoyl-α-L-arabinohexopyranosyl)oxy]-1,2,3,4,6,11-hexahydro-3,5,12-trihydroxy-3-methyl-6,11-dioxonaphthacen in Form orange-roter Kristalle vom Schmelzpunkt 244—247° (aus Aceton/Diäthyläther) erhalten. $[\alpha]_D^{20} = +273,8°$ (c = 0,1 % in Chloroform).

(B) Eine Lösung von 0,85 g (1 S)-cis-1-[(2,3,6-Trideoxy-3-trifluoracetamido-4-O-p-nitrobenzoyl-α-L-arabinohexopyranosyl)oxy]-1,2,3,4,6,11-hexahydro-3,5,12-trihydroxy-3-methyl-6,11-dioxonaphthacen in einem Gemisch von 50 ml Dichlormethan und 100 ml Methanol wurde bei 0° tropfenweise mit 0,1 N Natronlauge versetzt, bis eine Braun-Purpurfärbung bestehen blieb. Nach 30 Minuten zeigte die Dünnschichtchromatographie, daß kein Ausgangsmaterial mehr vorhanden war. Die Reaktion wurde durch Zusatz von Essigsäure bis zum Auftreten einer orangen Farbe abgestoppt. Das Gemisch wurde mit 250 ml Wasser verdünnt und mit viermal 100 ml Dichlormethan extrahiert. Die vereinigten Extrakte wurden über Natriumsulfat getrocknet und eingedampft und lieferten einen orangen Gummi, der aus Aceton/Diäthyläther kristallisiert (1 S)-cis-1-[(2,3,6-Trideoxy-3-trifluoracetamido-α-L-arabinohexopyranosyl)oxy]-1,2,3,4,6,11-hexahydro-3,5,12-trihydroxy-3-methyl-6,11-dioxonaphthacen in Form orange-roter Kristalle vom Schmelzpunkt 268—269° lieferte. $[\alpha]_D^{20} = +197,5°$ (c = 0,05 % in Chloroform).

(C) 565 mg der gemäß Absatz (B) erhaltenen Verbindung wurden in 50 ml Aceton gelöst und zu 60 ml 0,1 M Natronlauge gegeben. Nach 45 Minuten langem Rühren bei Raumtemperatur wurde die Lösung durch Zusatz von 0,1 N Salzsäure auf pH 8,5 eingestellt und wiederholt mit Dichlormethan, das 10% Äthanol enthielt, extrahiert bis die Extrakte vollständig farblos waren. Die vereinigten Extrakte wurden mit Wasser gewaschen, über Natriumsulfat getrocknet und zu einem roten Öl eingedampft. Dieses Öl wurde in 12 ml Dichlormethan und 3 ml Methanol gelöst und die Lösung wurde filtriert. Unter Umschwenken wurden 4 ml 0,25 N methanolische Salzsäure zugesetzt und die Lösung auf 8 ml konzentriert. Nach Ausfällen durch Zusatz von 75 ml trockenem Diäthyläther, Filtrieren, Waschen des Niederschlages mit trockenem Diäthyläther und Trocknen unter vermindertem Druck erhielt man 453 mg (1 S)-cis-1-[(3-Amino-2,3,6-trideoxy-α-L-arabinohexopyranosyl)oxy]-1,2,3,4,6,11-hexahydro-3,5,-

12-trihydroxy-3-methyl-6,11-dioxonaphthacen-hydrochlorid. Orange-rotes Pulver vom Schmelzpunkt 181—183° (Zersetzung); $[\alpha]_D^{20}$ = +242,6° (c = 0,5% in Methanol).

Beispiel 4

(A) In Analogie zu Beispiel 3 (A), wurde aus (1 S)-cis-3-Acetoxymethyl-1,2,3,4,6,11-hexahydro-1,-3,5,12-tetrahydroxy-6,11-dioxonaphthacen das (1 S)-cis-3-Acetoxymethyl-1-[(2,3,6-trideoxy-3-trifluoracetamido-4-O-p-nitrobenzoyl-α-L-arabinohexopyranosyl)oxy]-1,2,3,4,6,11-hexahydro-3,5,-12-trihydroxy-6,11-dioxonaphthacen in Form orange-roter Kristalle erhalten. Schmelzpunkt 262—263° (aus Aceton/Diäthyläther), $[\alpha]_D^{20}$ = +266,9° (c = 0,1% in Chloroform).

(B) Das gemäß dem vorangehenden Absatz erhaltene Produkt wurde wie in Beispiel 3 (B) behandelt, wobei die Reinigung durch Säulenchromatographie an Silicagel mit Äthylacetat/Hexan (1 : 1 Volumenteile) vorgenommen wurde. Man erhielt (1 S)-cis-3-Acetoxymethyl-1-[(2,3,6-trideoxy-3-trifluoracetamido-α-L-arabinohexopyranosyl)oxy]-1,2,3,4,6,11-hexahydro-3,5,12-trihydroxy-6,11-dioxonaphthacen in Form orange-roter Kristalle vom Schmelzpunkt 242—243° (aus Aceton/Diäthyläther), $[\alpha]_D^{20}$ = +193,7° (c = 0,1% in Chloroform).

(C) 0,8 g des so erhaltenen Produktes wurden in 100 ml Dichlormethan und 100 ml Methanol gelöst. Die Lösung wurde bis zum Auftreten einer tiefen Purpurfarbe mit 0,1 M Natronlauge versetzt, 5,5 Stunden bei Raumtemperatur gerührt und dann mit Essigsäure bis zum Auftreten einer orange-roten Färbung versetzt. Die erhaltene Lösung wurde mit 250 ml Wasser verdünnt und mit viermal 100 ml Dichlormethan extrahiert. Die Extrakte wurden über Natriumsulfat getrocknet und eingedampft und lieferten einen orangen Feststoff. Kristallisation aus Aceton/Diäthyläther lieferte 0,63 g (1 S)-cis-1-[(2,3,-6-Trideoxy-3-trifluoracetamido-α-L-arabinohexopyranosyl)oxy]-1,2,3,4,6,11-hexahydro-3,5,12-trihydroxy-3-hydroxymethyl-6,11-dioxonaphthacen in Form orange-roter Kristalle vom Schmelzpunkt 237—239°; $[\alpha]_D^{20}$ = +236,9° (c = 0,1% in Aceton).

(D) Das gemäß dem vorstehenden Absatz erhaltene Produkt wurde in Analogie zu Beispiel 3 (C) behandelt und lieferte (1 S)-cis-1-[(3-Amino-2,3,6-trideoxy-α-L-arabinohexopyranosyl)oxy]-1,2,3,4,6,-11-hexahydro-3,5,12-trihydroxy-3-hydroxymethyl-6,11-dioxonaphthacen-hydrochlorid in Form eines orangen Pulvers vom Schmelzpunkt 189—193° (Zersetzung); $[\alpha]_D^{20}$ = +215,8° (c = 0,05% in Methanol).

Beispiel 5

(A) In Analogie zu Beispiel 1 (A) wurde aus (1 S)-cis-3-Äthyl-1,2,3,4,6,11-hexahydro-1,3,5,12-tetrahydroxy-6,11-dioxonaphthacen das (1 S)-cis-1-[(2,3,6-Trideoxy-3-trifluoracetamido-4-O-p-nitrobenzoyl-α-L-lyxohexopyranosyl)oxy]-3-äthyl-1,2,3,4,6,11-hexahydro-3,5,12-trihydroxy-6,11-dioxonaphthacen in Form roter Kristalle erhalten. Schmelzpunkt 181,5—182,5° (aus Äthylacetat/Petroläther [60—80°]), $[\alpha]_D^{20}$ = −98,4° (c = 0,1% in Dioxan).

(B) Das so erhaltene Produkt wurde gemäß Beispiel 1 (B) behandelt und lieferte (1 S)-cis-1-[(2,3,6-Trideoxy-3-trifluoracetamido-α-L-lyxohexopyranosyl)oxy]-3-äthyl-1,2,3,4,6,11-hexahydro-3,5,12-trihydroxy-6,11-dioxonaphthacen in Form oranger Kristalle vom Schmelzpunkt 241—243°; $[\alpha]_D^{20}$ = +188,6° (c = 0,1% in Dioxan).

(C) Das so erhaltene Produkt wurde gemäß Beispiel 1 (D) behandelt und lieferte (1 S)-cis-1-[(3-Amino-2,3,6-trideoxy-α-L-lyxohexopyranosyl)oxy]-3-äthyl-1,2,3,4,6,11-hexahydro-3,5,12-trihydroxy-6,11-dioxonaphthacen-hydrochlorid in Form orange-roter Kristalle vom Schmelzpunkt 174—176°; $[\alpha]_D^{20}$ = +175,8° (c = 0,1% in Methanol).

Das als Ausgangsmaterial verwendete (1 S)-cis-3-Äthyl-1,2,3,4,6,11-hexahydro-1,3,5,12-tetrahydroxy-6,11-dioxonaphthacen kann wie folgt hergestellt werden:

(a) In Analogie zu Beispiel 1 (b) wurde aus (1 S)-cis-3-Äthyl-1,2,3,4,5,12-hexahydro-5,12-dioxo-1,3-naphthacendiyl-benzolboronat das (1 S)-cis-5,12-Diacetoxy-3-äthyl-1,2,3,4-tetrahydro-1,3-naphthacendiyl-benzolboronat erhalten, das ohne weitere Reinigung im nächsten Schritt verwendet wurde.

(b) Oxidation der vorstehend genannten Verbindung in Analogie zu Beispiel 1 (c) lieferte (1 S)-cis-5,-12-Diacetoxy-3-äthyl-1,2,3,4,6,11-hexahydro-6,11-dioxo-1,3-naphthacendiyl-benzolboronat, das im nächsten Schritt ohne weitere Reinigung eingesetzt wurde.

(c) Behandlung der vorstehend genannten Dioxoverbindung mit Bortrichlorid in Analogie zu Beispiel 1 (d) lieferte (1 S)-cis-3-Äthyl-1,2,3,4,6,11-hexahydro-5,12-dihydroxy-6,11-dioxo-1,3-naphthacendiyl-benzolboronat, das im nächsten Schritt ohne weitere Reinigung eingesetzt wurde.

(d) In Analogie zu Beispiel 1 (e) wurde aus der vorstehend genannten Dihydroxyverbindung das (1 S)-cis-3-Äthyl-1,2,3,4,6,11-hexahydro-1,3,5,12-tetrahydroxy-6,11-dioxonaphthacen vom Schmelzpunkt 179—183°; $[\alpha]_D^{20}$ = +136,2° (c = 0,1% in Dioxan) erhalten.

Das im Schritt (a) dieses Beispiels als Ausgangsmaterial verwendete (1 S)-cis-3-Äthyl-1,2,3,4,5,12-hexahydro-5,12-dioxo-1,3-naphthacendiyl-benzolboronat kann wie folgt hergestellt werden:

(i) 12,95 g einer 50%igen Dispersion von Natriumhydrid in Mineralöl wurden unter Rühren und in

Stickstoffatmosphäre zu 152 ml trockenem Dimethylsulfoxid gegeben. Das Gemisch wurde bei 70° gerührt bis die Wasserstoffentwicklung aufhörte. Nach Abkühlen auf 0° wurden 152 ml trockenes Tetrahydrofuran zugesetzt. Danach wurden im Verlauf von 10 Minuten tropfenweise eine Lösung von 20,03 g (S)-1',2',3',4'-Tetrahydro-2'-hydroxy-5',8'-dimethoxyspiro[1,3-dithiolan-2,4'-naphthalin]-2'-carbonsäure-methylester (hergestellt wie in Beispiel 1 (vi)) in 152 ml trockenem Tetrahydrofuran gegeben. Nach 15 Minuten langem Rühren bei 0° wurde das Gemisch in 1000 ml Wasser gegossen und durch Zusatz von Salzsäure auf pH 3 eingestellt. Die Lösung wurde mit viermal 400 ml Dichlormethan extrahiert, die Extrakte mit 650 ml Wasser gewaschen, über Magnesiumsulfat getrocknet und eingedampft. Das in Form eines orangen Öls so erhaltene β-Ketosulfoxid wurde in einem Gemisch von 985 ml Tetrahydrofuran und 98,5 ml Wasser gelöst. Die Lösung wurde unter Stickstoff gerührt und auf 12° abgekühlt. Danach wurde aus 15,2 g Al-Folie hergestelltes Aluminiumamalgam zugesetzt und das Gemisch wurde 2 Stunden bei 12–15° gerührt. Danach wurde filtriert und das Tetrahydrofuran abgedampft. Der Rückstand wurde in 530 ml Dichlormethan gelöst, die Lösung mit zweimal 1000 ml Wasser gewaschen, über Magnesiumsulfat getrocknet und eingedampft. Der so erhaltene crèmefarbene Feststoff wurde aus Dichlormethan/Diäthyläther umkristallisiert und lieferte 11,55 g (S)-3'-Acetyl-1',2',3',4'-tetrahydro-3'-hydroxy-5',8'-dimethoxyspiro[1,3-dithiolan-2,1'-naphthalin] in Form farbloser Kristalle vom Schmelzpunkt 175–178°; $[\alpha]_D^{20} = -25,3°$ (c = 0,5% in Chloroform).

(ii) Eine Suspension von 16,48 g der vorstehend genannten Spiroverbindung in 2450 ml trockenem Tetrahydrofuran wurde mit 3,59 g Natriumborhydrid versetzt. Das Gemisch wurde unter Stickstoff bei Raumtemperatur 4 Stunden gerührt. Danach wurde das Lösungsmittel abgedampft und der weiße Rückstand auf 0° gekühlt. Nach Zusatz von 1500 ml 5%iger Ammoniumchloridlösung wurde das Gemisch bei Raumtemperatur gerührt, bis keine Gasentwicklung mehr auftrat. Die Lösung wurde mit dreimal 500 ml Äthylacetat extrahiert, die Extrakte mit 1000 ml Wasser, über Magnesiumsulfat getrocknet und eingedampft. Man erhielt 17,65 g (3'S)-1',2',3',4'-Tetrahydro-3'-hydroxy-3'-(1-hydroxyäthyl)-5',8'-dimethoxyspiro[1,3-dithiolan-2,1'-naphthalin] in Form eines farblosen Gummis, der ohne weitere Reinigung verwendet wurde.

(iii) 17,65 g der vorstehend genannten Hydroxyäthylverbindung wurden in 380 ml trockenem Pyridin gelöst. Die Lösung wurde bei 0° unter Rühren und Stickstoffatmosphäre mit einer Lösung von 5,55 g Methansulfonylchlorid in 50 ml trockenem Pyridin versetzt und 18,5 Stunden bei 4° stehen gelassen. Danach wurde das Pyridin abgedampft, 500 ml Wasser und 250 ml Äthylacetat zugesetzt und das Gemisch 10 Minuten lang gerührt. Die wäßrige Phase wurde mit dreimal 250 ml Äthylacetat extrahiert. Die vereinigten Äthylacetatextrakte wurden mit zweimal 500 ml Wasser, zweimal 500 ml 2 N Salzsäure und 500 ml gesättigter Natriumhydrogencarbonatlösung gewaschen, über Magnesiumsulfat getrocknet und eingedampft. Man erhielt 20,74 g 3',4'-Dihydro-3'-hydroxy-5',8'-dimethoxy-α-methylspiro[1,3-dithiolan-2,1'(2'H)-naphthalin]-3'-ylmethyl-methansulfonat in Form eines farblosen Gummis, der ohne weitere Reinigung verwendet wurde.

(iv) Ein Gemisch von 3,68 g Lithiumaluminiumhydrid und 200 ml trockenem Tetrahydrofuran wurde unter Stickstoff gerührt und mit einer Lösung von 20,74 g des vorstehend erwähnten Methansulfonats in 300 ml trockenem Tetrahydrofuran versetzt. Das Gemisch wurde 50 Minuten lang unter Rühren zum Rückfluß erhitzt und anschließend auf 0° gekühlt. Danach wurden 110 ml 10%ige Ammoniumchloridlösung zugesetzt und das Tetrahydrofuran abgedampft. Der Rückstand wurde mit 1000 ml 2 N Salzsäure versetzt und mit dreimal 500 ml Äthylacetat extrahiert. Die Äthylacetatextrakte wurden mit 500 ml gesättigter Natriumhydrogencarbonatlösung gewaschen, über Magnesiumsulfat getrocknet und eingedampft. Man erhielt 15,31 g (S)-3'-Äthyl-1',2',3',4'-tetrahydro-3'-hydroxy-5',8'-dimethoxyspiro[1,3-dithiolan-2,1'-naphthalin] in Form eines farblosen Gummis, der ohne weitere Reinigung verwendet wurde.

(v) In Analogie zu Beispiel 1 (ix) wurden aus 15,31 g der im vorhergehenden Absatz erhaltenen Verbindung 7,38 g (S)-3-Äthyl-1,2,3,4-tetrahydro-3-hydroxy-5,8-dimethoxy-1-oxo-naphthalin in Form eines weißen Pulvers vom Schmelzpunkt 132–134° erhalten.

(vi) Eine Lösung von 7,38 g (S)-3-Äthyl-1,2,3,4-tetrahydro-3-hydroxy-5,7-dimethoxy-1-oxo-naphthalin in 490 ml trockenem Tetrahydrofuran wurden unter Rühren mit 1,73 g Lithiumborhydrid versetzt. Das Gemisch wurde 70 Minuten bei Raumtemperatur gerührt und dann zur Entfernung des Tetrahydrofurans eingedampft. Der Rückstand wurde mit 250 ml 10%iger Ammonchloridlösung versetzt und mit dreimal 250 ml Äthylacetat extrahiert. Die Extrakte wurden mit 250 ml Wasser gewaschen und über Magnesiumsulfat getrocknet. Nach Filtrieren wurde das Filtrat mit 3,86 g Benzolboronsäure und 0,6 ml Essigsäure versetzt. Die Lösung wurde 45 Minuten unter Rühren zum Rückfluß erhitzt und dann eingedampft. Der Rückstand wurde in 650 ml Toluol gelöst und die Lösung mit 1,93 g Benzolboronsäure und 0,197 g p-Toluolsulfonsäure versetzt. Das Gemisch wurde 16,5 Stunden bei Raumtemperatur gerührt, mit zweimal 250 ml 10%iger Kaliumhydrogencarbonatlösung gewaschen, getrocknet und eingedampft. Man erhielt 9,18 g (1 S)-cis-3-Äthyl-1,2,3,4-tetrahydro-5,8-dimethoxy-1,3-naphthalindiyl-benzolboronat in Form weißer Kristalle vom Schmelzpunkt 132–133°; $[\alpha]_D^{20} = +56,0°$ (c = 0,5% in Chloroform).

(vii) Eine Lösung von 9,15 g des vorstehend erhaltenen Benzolboronats in 400 ml Acetonitril wurde unter Rühren mit einer Lösung von 29,83 g Ammoniumcernitrat in 400 ml Wasser im Verlauf von 5 Minuten versetzt. Das Gemisch wurde 5 Minuten bei Raumtemperatur gerührt und dann in 2200 ml

Wasser gegossen. Das erhaltene Gemisch wurde mit fünfmal 270 ml Dichlormethan extrahiert, der Extrakt mit 550 ml Wasser gewaschen, getrocknet und eingedampft. Man erhielt 8,23 g (1 S)-cis-3-Äthyl-1,2,3,4-tetrahydro-5,8-dioxo-naphthalindiyl-benzolboronat in Form eines gelben Gummis, der ohne weitere Reinigung verwendet wurde.

(viii) 8,23 g des im vorhergehenden Absatz erhaltenen Produkts und 7,48 g trans-1,2-Diacetoxy-1,2-dihydrobenzocyclobuten wurden in 500 ml Xylol gelöst und 195 Minuten bei 140° gerührt. Danach wurde das Xylol abgedampft und der Rückstand mit 200 ml Äther gerührt. Nach Filtration wurden 7,96 g (1 S)-cis-3-Äthyl-1,2,3,4,5,12-hexahydro-5,12-dioxo-1,3-naphthacendiyl-benzolboronat in Form eines hellgelben Feststoffes vom Schmelzpunkt 225–226° erhalten. $[\alpha]_D^{20} = +143{,}3°$ (c = 0,1 % in Chloroform).

**Patentansprüche für die Vertragsstaaten: BE, CH, DE, FR, GB, IT, LI, LU, NL, SE**

1. Verbindungen der allgemeinen Formel

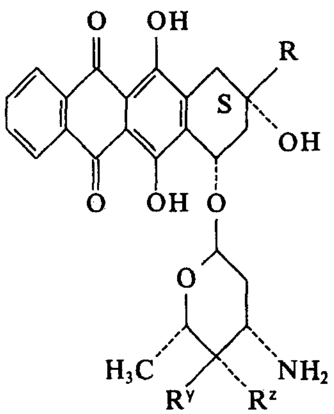

(I)

worin R nieder-Alkyl oder Carboxy oder eine Gruppe der Formel

$$-(CH_2)_n-OY \qquad (a)$$

darstellt, wobei n 1 oder 2, Y Wasserstoff oder Alkyl und $R^y$ und $R^z$ jeweils Wasserstoff oder einer der Reste $R^y$ und $R^z$ Wasserstoff und der andere Hydroxy bedeuten, und pharmazeutisch anwendbare Säureadditionssalze davon.

2. Verbindungen gemäß Anspruch 1, in denen R nieder-Alkyl oder eine Gruppe der Formel (a), wobei Y Wasserstoff ist, darstellt.

3. Verbindungen gemäß Anspruch 1 oder 2, in denen ein Rest $R^y$ und $R^z$ Wasserstoff und der andere Hydroxy ist.

4. (1 S)-cis-1-[(3-Amino-2,3,6-trideoxy-α-L-lyxohexopyranosyl)oxy]-1,2,3,4,6,11-hexahydro-3,5,-12-trihydroxy-3-hydroxymethyl-6,11-dioxonaphthacen.

5. (1 S)-cis-1-[(3-Amino-2,3,6-trideoxy-α-L-lyxohexopyranosyl)oxy]-1,2,3,4,6,11-hexahydro-3,5,-12-trihydroxy-3-methyl-6,11-dioxonaphthacen.

6. (1 S)-cis-1-[(3-Amino-2,3,6-trideoxy-α-L-arabinohexopyranosyl)oxy]-1,2,3,4,6,11-hexahydro-3,5,12-trihydroxy-3-methyl-6,11-dioxonaphthacen, (1 S)-cis-1-[(3-Amino-2,3,6-trideoxy-α-L-arabinohexopyranosyl)oxy]-1,2,3,4,6,11-hexahydro-3,5,12-trihydroxy-3-hydroxymethyl-6,11-dioxonaphthacen und (1 S)-cis-1-[(3-Amino-2,3,6-trideoxy-α-L-lyxohexopyranosyl)oxy]-3-äthyl-1,2,3,4,-6,11-hexahydro-3,5,12-trihydroxy-6,11-dioxonaphthacen.

7. Verbindungen der Formel I von Anspruch 1 als Wirkstoffe in Medikamenten.

8. Verbindungen der Formel I von Anspruch 1 bei der Behandlung maligner Tumore.

9. Verfahren zur Herstellung von Verbindungen der Formel I von Anspruch 1, dadurch gekennzeichnet, daß man eine Verbindung der Formel

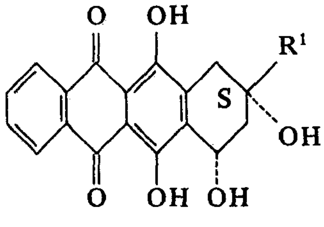

(II)

worin $R^1$ nieder-Alkyl oder eine veresterte Carboxygruppe oder eine Gruppe der Formel

$$-(CH_2)_n-OY' \qquad (a')$$

worin n die obige Bedeutung hat und Y' Alkyl oder Acyl ist, darstellt, mit einer Verbindung der Formel

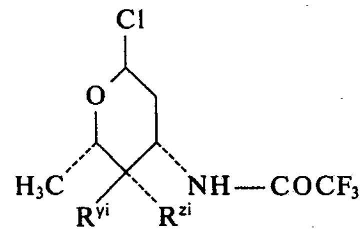

(III)

worin $R^{yi}$ und $R^{zi}$ jeweils Wasserstoff oder einer der Reste $R^{yi}$ und $R^{zi}$ Wasserstoff und der andere p-Nitrobenzoyloxy darstellen, umsetzt, die Schutzgruppe(n) aus dem Reaktionsprodukt abspaltet und gewünschtenfalls die erhaltene Verbindung der Formel I in ein pharmazeutisch anwendbares Säureadditionssalz überführt.

10. Pharmazeutische Präparate, enthaltend eine Verbindung der Formel I von Anspruch 1.

**Patentansprüche für den Vertragsstaat: AT**

1. Verfahren zur Herstellung von Verbindungen der allgemeinen Formel

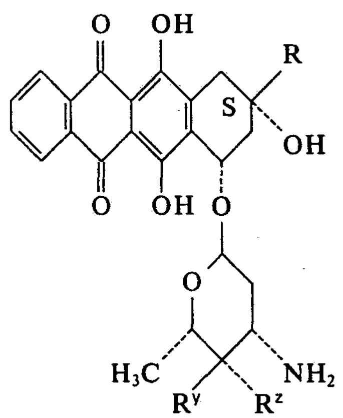

(I)

worin R nieder-Alkyl oder Carboxy oder eine Gruppe der Formel

$$-(CH_2)_n-OY \qquad (a)$$

darstellt, wobei n 1 oder 2, Y Wasserstoff oder Alkyl und $R^y$ und $R^z$ jeweils Wasserstoff oder einer der Reste $R^y$ und $R^z$ Wasserstoff und der andere Hydroxy bedeuten, und pharmazeutisch anwendbare Säureadditionssalze davon, dadurch gekennzeichnet, daß man eine Verbindung der Formel

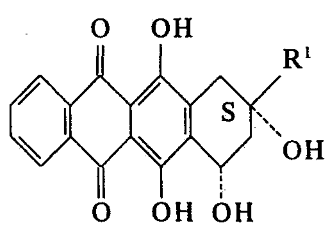

(II)

worin $R^1$ nieder-Alkyl oder eine veresterte Carboxygruppe oder eine Gruppe der Formel

$$-(CH_2)_n-OY' \qquad (a')$$

worin n die obige Bedeutung hat und Y' Alkyl oder Acyl ist, darstellt, mit einer Verbindung der Formel

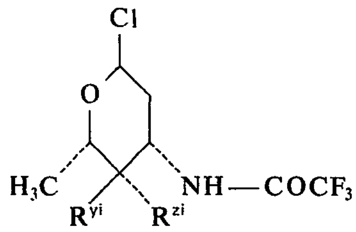

(III)

worin $R^{y'}$ und $R^{zi}$ jeweils Wasserstoff oder einer der Reste $R^{yi}$ und $R^{zi}$ Wasserstoff und der andere p-Nitrobenzoyloxy darstellen, umsetzt, die Schutzgruppe(n) aus dem Reaktionsprodukt abspaltet und gewünschtenfalls die erhaltene Verbindung der Formel I in ein pharmazeutisch anwendbares Säureadditionssalz überführt.

2. Verfahren gemäß Anspruch 1, dadurch gekennzeichnet, daß man Verbindungen der Formel I, in denen R nieder-Alkyl oder eine Gruppe der Formel (a), wobei Y Wasserstoff ist, darstellt.

3. Verfahren gemäß den Ansprüchen 1 oder 2, dadurch gekennzeichnet, daß man Verbindungen der Formel I, in denen ein Rest $R^y$ und $R^z$ Wasserstoff und der andere Hydroxy ist, herstellt.

4. Verfahren gemäß den Ansprüchen 1—3, dadurch gekennzeichnet, daß man (1 S)-cis-1-[(3-Amino-2,3,6-trideoxy-$\alpha$-L-lyxohexopyranosyl)oxy]-1,2,3,4,6,11-hexahydro-3,5,12-trihydroxy-3-hydroxymethyl-6,11-dioxonaphthacen herstellt.

5. Verfahren gemäß den Ansprüchen 1—3, dadurch gekennzeichnet, daß man (1 S)-cis-1-[(3-Amino-2,3,6-trideoxy-$\alpha$-L-lyxohexopyranosyl)oxy]-1,2,3,4,6,11-hexahydro-3,5,12-trihydroxy-3-methyl-6,-11-dioxonaphthacen herstellt.

6. Verfahren gemäß den Ansprüchen 1—3, dadurch gekennzeichnet, daß man (1 S)-cis-1-[(3-Amino-2,3,6-trideoxy-$\alpha$-L-arabinohexopyranosyl)oxy]-1,2,3,4,6,11-hexahydro-3,5,12-trihydroxy-3-methyl-6,11-dioxonaphthacen, (1 S)-cis-1-[(3-Amino-2,3,6-trideoxy-$\alpha$-L-arabinohexopyranosyl)oxy]-1,2,3,4,6,11-hexahydro-3,5,12-trihydroxy-3-hydroxymethyl-6,11-dioxonaphthacen und (1 S)-cis-1-[(3-Amino-2,3,6-trideoxy-$\alpha$-L-lyxohexopyranosyl)oxy]-3-äthyl-1,2,3,4,6,11-hexahydro-3,5,12-trihydroxy-6,11-dioxonaphthacen herstellt.

**Claims for the Contracting States: BE, CH, DE, FR, GB, IT, LI, LU, NL, SE**

1. Compounds of the general formula

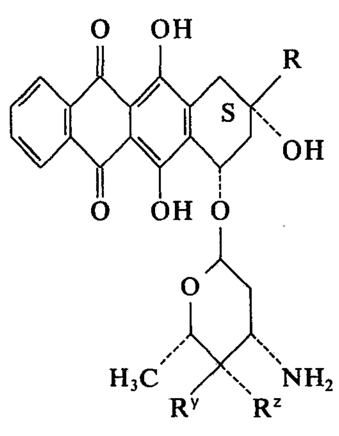

(I)

wherein R represents lower-alkyl or carboxy or a group of the formula

$$-(CH_2)_n-OY \qquad (a)$$

in which n signifies 1 or 2, Y signifies hydrogen or alkyl and $R^y$ and $R^z$ each signify hydrogen or one of the residues $R^y$ and $R^z$ signifies hydrogen and the other signifies hydroxy, and pharmaceutically acceptable acid addition salts thereof.

2. Compounds in accordance with claim 1, in which R represents lower-alkyl or a group of formula (a) in which Y is hydrogen.

3. Compounds in accordance with claim 1 or 2, in which one residue $R^y$ and $R^z$ is hydrogen and the other is hydroxy.

4. (1 S)-cis-1-[(3-Amino-2,3,6-trideoxy-α-L-lyxohexopyranosyl)oxy]-1,2,3,4,6,11-hexahydro-3,5,-12-trihydroxy-3-hydroxymethyl-6,11-dioxonaphthacene.

5. (1 S)-cis-1-[(3-Amino-2,3,6-trideoxy-α-L-lyxohexopyranosyl)oxy]-1,2,3,4,6,11-hexahydro-3,5,-12-trihydroxy-3-methyl-6,11-dioxonaphthacene.

6. (1 S)-cis-1-[(3-Amino-2,3,6-trideoxy-α-L-arabinohexopyranosyl)oxy]-1,2,3,4,6,11-hexahydro-3,5,12-trihydroxy-3-methyl-6,11-dioxonaphthacene, (1 S)-cis-1-[(3-amino-2,3,6-trideoxy-α-L-arabinohexopyranosyl)oxy]-1,2,3,4,6,11-hexahydro-3,5,12-trihydroxy-3-hydroxymethyl-6,11-dioxonaphthacene and (1 S)-cis-1-[(3-amino-2,3,6-trideoxy-α-L-lyxohexopyranosyl)oxy]-3-ethyl-1,2,3,4,-6,11-hexahydro-3,5,12-trihydroxy-6,11-dioxonaphthacene.

7. Compounds of formula I of claim 1 as active substances in medicaments.

8. Compounds of formula I of claim 1 in the treatment of malign tumours.

9. A process for the manufacture of compounds of formula I of claim 1, characterized by reacting a compound of the formula

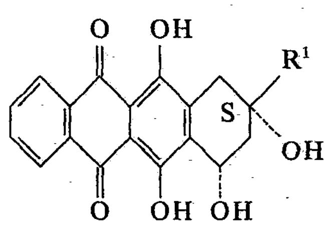

(II)

wherein $R^1$ represents lower-alkyl or an esterified carboxy group or a group of the formula

$$—(CH_2)_n—OY' \qquad (a')$$

wherein n has the above significance and Y' is alkyl or acyl, with a compound of the formula

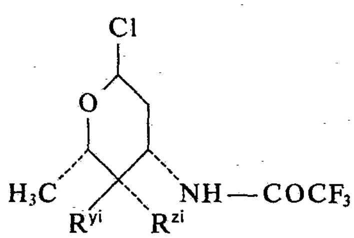

(III)

wherein $R^{yi}$ and $R^{zi}$ each represent hydrogen or one of the residues $R^{yi}$ and $R^{zi}$ represents hydrogen and the other represents p-nitrobenzoyloxy, cleaving off the protecting group(s) from the reaction product and, if desired, converting the compound of formula I obtained into a pharmaceutically acceptable acid addition salt.

10. Pharmaceutical preparations, containing a compound of formula I of claim 1.

**Claims for the Contracting State: AT**

1. A process for the manufacture of compounds of the general formula

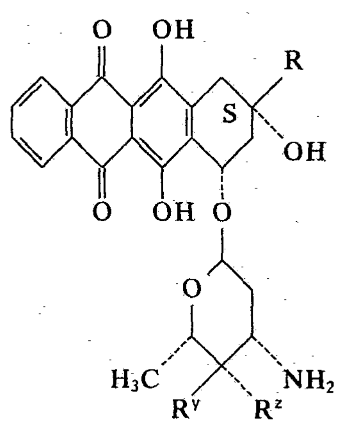

(I)

wherein R represents lower-alkyl or carboxy or a group of the formula

$$-(CH_2)_n-OY \qquad (a)$$

in which n signifies 1 or 2, Y signifies hydrogen or alkyl and $R^y$ and $R^z$ each signify hydrogen or one of the residues $R^y$ and $R^z$ signifies hydrogen and the other signifies hydroxy,
and pharmaceutically acceptable acid addition salts thereof, characterized by reacting a compound of the formula

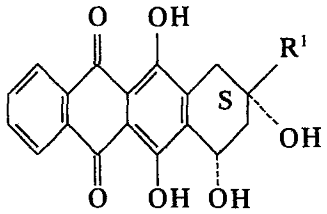

(II)

wherein $R^1$ represents lower-alkyl or an esterified carboxy group or a group of the formula

$$-(CH_2)_n-OY' \qquad (a')$$

wherein n has the above significance and Y' is alkyl or acyl,
with a compound of the formula

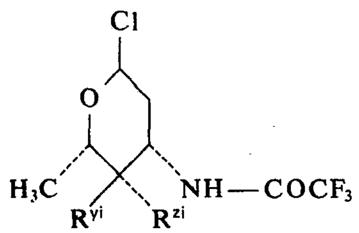

(III)

wherein $R^{yi}$ and $R^{zi}$ each represent hydrogen or one of the residues $R^{yi}$ and $R^{zi}$ represents hydrogen and the other represents p-nitrobenzoyloxy,
cleaving off the protecting group(s) from the reaction group and, if desired, converting the compound of formula I obtained into a pharmaceutically acceptable acid addition salt.

2. A process in accordance with claim 1, characterized in that compounds of formula I in which R represents lower-alkyl or a group of formula (a) in which Y is hydrogen are manufactured.

3. A process in accordance with claims 1 or 2, characterized in that compounds of formula I in which one residue $R^y$ and $R^z$ is hydrogen and the other is hydroxy are manufactured.

4. A process in accordance with claims 1—3, characterized in that (1 S)-cis-1-[(3-amino-2,3,6-trideoxy-α-L-lyxohexopyranosyl)oxy]-1,2,3,4,6,11-hexahydro-3,5,12-trihydroxy-3-hydroxymethyl-6,-11-dioxonaphthacene is manufactured.

5. A process in accordance with claims 1—3, characterized in that (1 S)-cis-1-[(3-amino-2,3,6-trideoxy-α-L-lyxohexopyranosyl)oxy]-1,2,3,4,6,11-hexahydro-3,5,12-trihydroxy-3-methyl-6,11-dioxonaphthacene is manufactured.

6. A process in accordance with claims 1—3, characterized in that (1 S)-cis-1-[(3-amino-2,3,6-trideoxy-α-L-arabinohexopyranosyl)oxy]-1,2,3,4,6,11-hexahydro-3,5,12-trihydroxy-3-methyl-6,11-dioxonaphthacene, (1 S)-cis-1-[(3-amino-2,3,6-trideoxy-α-L-arabinohexopyranosyl)oxy]-1,2,3,4,6,-11-hexahydro-3,5,12-trihydroxy-3-hydroxymethyl-6,11-dioxonaphthacene and (1 S)-cis-1-[(3-amino-2,3,6-trideoxy-α-L-lyxohexopyranosyl)oxy]-3-ethyl-1,2,3,4,6,11-hexahydro-3,5,12-trihydroxy-6,11-dioxonaphthacene are manufactured.

**Revendications pour les Etats Contractants: BE, CH, DE, FR, GB, IT, LI, LU, NL, SE**

1. Composés de formule générale

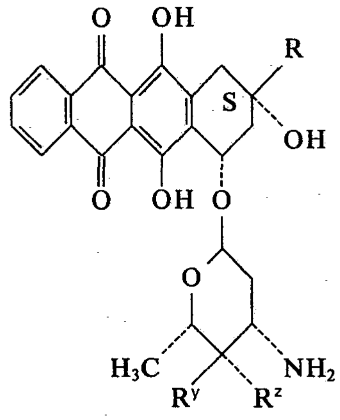

(I)

dans laquelle R représente un groupe alkyle inférieur ou carboxy ou un groupe de formule

$$—(CH_2)_n—OY \qquad (a)$$

n est égal à 1 ou 2, Y représente l'hydrogène ou un groupe alkyle et $R^y$ et $R^z$ représentent tous deux l'hydrogène ou bien l'un des symboles $R^y$ et $R^z$ représente l'hydrogène et l'autre un groupe hydroxy, et leurs sels formés par addition avec des acides acceptables pour l'usage pharmaceutique.

2. Composés selon la rev. 1, dans lesquels R représente un groupe alkyle inférieur ou un groupe de formule (a) dans laquelle Y représente l'hydrogène.

3. Composés selon la rev. 1 ou 2, dans lesquels l'un des symboles $R^y$ et $R^z$ représente l'hydrogène et l'autre un groupe hydroxy.

4. Le (1 S)-cis-1[(3-amino-2,3,6-tridésoxy-alpha-L-lyxohexopyrannosyl)-oxy]-1,2,3,4,6,11-hexahydro-3,5,12-trihydroxy-3-hydroxyméthyl-6,11-dioxonaphtacène.

5. Le (1 S)-cis-1-[(3-amino-2,3,6-tridésoxy-alpha-L-lyxohexopyrannosyl)-oxy]-1,2,3,4,6,11-hexahydro-3,5,12-trihydroxy-3-méthyl-6,11-dioxonaphtacène.

6. Le (1 S)-cis-1-[(3-amino-2,3,6-tridésoxy-alpha-L-arabinohexopyrannosyl)-oxy]-1,2,3,4,6,11-hexahydro-3,5,12-trihydroxy-3-méthyl-6,12-dioxonaphtacène, le (1 S)-cis-1-[(3-amino-2,3,6-tridésoxy-alpha-L-arabinohexopyrannosyl)-oxy]-1,2,3,4,6,11-hexahydro-3,5,12-trihydroxy-3-hydroxyméthyl-6,11-dioxonaphtacène et le (1 S)-cis-1-[(3-amino-2,3,6-tridésoxy-alpha-L-lyxohexopyrannosyl)-oxy]-3-éthyl-1,2,3,4,6,11-hexahydro-3,5,12-trihydroxy-6,11-dioxonaphtacène.

7. Composés de formule I selon la rev. 1, en tant que substances actives dans des médicaments.

8. Composés de formule I selon la rev. 1, pour le traitement de tumeurs malignes.

9. Procédé de préparation des composés de formule I selon la rev. 1, caractérisé en ce que l'on fait réagir un composé de formule

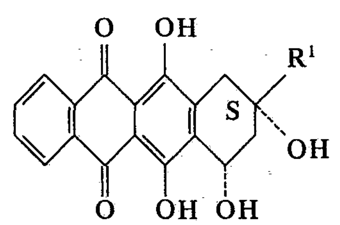

(II)

dans laquelle $R^1$ représente un groupe alkyle inférieur ou un groupe carboxy estérifié ou un groupe de formule

$$—(CH_2)_n—OY' \qquad (a')$$

dans laquelle n a la signification indiquée ci-dessus et Y' représente un groupe alkyle ou acyle, avec un composé de formule

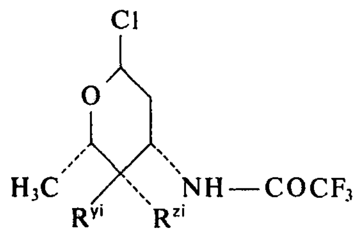

(III)

dans laquelle $R^{yi}$ et $R^{zi}$ représentent tous deux l'hydrogène ou bien l'un des symboles $R^{yi}$ et $R^{zi}$ représente l'hydrogène et l'autre le groupe p-nitrobenzoyloxy, on élimine du produit de réaction le ou les groupes protecteurs et, si on le désire, on convertit le composé de formule I ainsi obtenu en un sel par addition avec un acide acceptable pour l'usage pharmaceutique.

10. Compositions pharmaceutiques contenant un composé de formule I de la rev. 1.

**Revendications pour l'État contractant: AT**

1. Procédé de préparation des composés de formule générale

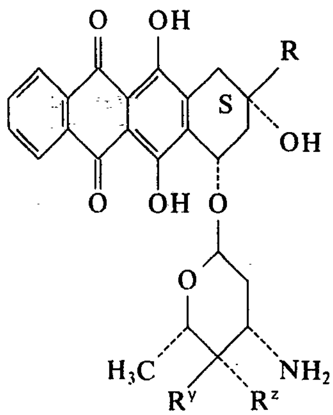

(I)

dans laquelle R représente un groupe alkyle inférieur ou carboxy ou un groupe de formule

$$-(CH_2)_n-OY \qquad (a)$$

dans laquelle n est égal à 1 ou 2, Y représente l'hydrogène ou un groupe alkyle et $R^y$ et $R^z$ représentent tous deux l'hydrogène ou bien l'un des symboles $R^y$ et $R^z$ représente l'hydrogène et l'autre un groupe hydroxy,
et de leurs sels formés par addition avec des acides acceptables pour l'usage pharmaceutique, caractérisé en ce que l'on fait réagir un composé de formule

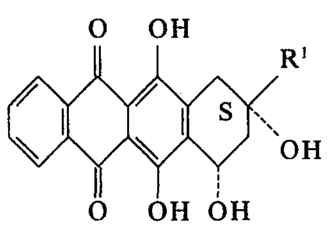

(II)

dans laquelle $R^1$ représente un groupe alkyle inférieur ou un groupe carboxy estérifié ou un groupe de formule

$$-(CH_2)_n-OY' \qquad (a')$$

dans laquelle n a la signification indiquée ci-dessus et Y' représente un groupe alkyle ou acyle, avec un composé de formule

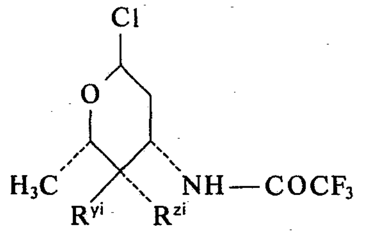

(III)

dans laquelle $R^{yi}$ et $R^{zi}$ représentent tous deux l'hydrogène ou bien l'un des symboles $R^{yi}$ et $R^{zi}$ représente l'hydrogène et l'autre le groupe p-nitrobenzoyloxy,
on élimine du produit de réaction le ou les groupes protecteurs et, si on le désire, on convertit le composé obtenu répondant à la formule I en un sel par addition avec un acide acceptable pour l'usage pharmaceutique.

2. Procédé selon la rev. 1, caractérisé en ce que l'on prépare des composés de formule I dans laquelle R représente un groupe alkyle inférieur ou un groupe de formule (a) dans laquelle Y représente l'hydrogène.

3. Procédé selon les rev. 1 ou 2, caractérisé en ce que l'on prépare des composés de formule I dans laquelle l'un des symboles $R^y$ et $R^z$ représente l'hydrogène et l'autre un groupe hydroxy.

4. Procédé selon les rev. 1 à 3, caractérisé en ce que l'on prépare le (1 S)-cis-1[(3-amino-2,3,6-tridésoxy-alpha-L-lyxohexopyrannosyl)-oxy]-1,2,3,4,6,11-hexahydro-3,5,12-trihydroxy-3-hydroxyméthyl-6,11-dioxonaphtacène.

5. Procédé selon les rev. 1 à 3, caractérisé en ce que l'on prépare le (1 S)-cis-1-[(3-amino-2,3,6-tridésoxy-alpha-L-lyxohexopyrannosyl)-oxy]-1,2,3,4,6,11-hexahydro-3,5,12-trihydroxy-3-méthyl-6,11-dioxonaphtacène.

6. Procédé selon les rev. 1 à 3, caractérisé en ce que l'on prépare le (1 S)-cis-1-[(3-amino-2,3,6-tridésoxy-alpha-L-arabinohexopyrannosyl)-oxy]-1,2,3,4,6,11-hexahydro-3,5,12-trihydroxy-3-méthyl-6,11-dioxonaphtacène, le (1 S)-cis-1-[(3-amino-2,3,6-tridésoxy - alpha - L - arabinohexopyrannosyl)-oxy] - 1,2,3,4,6,11 - hexahydro - 3,5,12 - trihydroxy - 3 - hydroxyméthyl-6,11-dioxonaphtacène et le (1 S)-cis-1-[(3-amino-2,3,6-tridésoxy-alpha-L-lyxohexopyrannosyl)-oxy]-3-éthyl-1,2,3,4,6,11-hexahydro-3,5,12-trihydroxy-6,11-dioxonaphtacène.